# EUROPEAN PATENT SPECIFICATION

(11) **EP 2 366 396 B1**
(45) Date of publication and mention of the grant of the patent: **07.08.2013**
(21) Application number: 10011221.8
(22) Date of filing: 27.01.2004
(51) Int. Cl.: A61K 31/7076

(54) **Myocardial perfusion imaging using adenosine receptor agonists**
Myokardiale Perfusionsbildgebung mittels Adenosinrezeptorantagonisten
Imagerie de perfusion du myocarde utilisant des agonistes de récepteur d'adénosine

(43) Date of publication of application: 21.09.2011
(62) Divisional of application: 04705715.3
(73) Proprietor: Gilead Sciences, Inc., Foster City, CA 94404 (US)
(72) Inventor: Belardinelli, Luiz, Palo Alto CA 94306 (US); Rosner, Mitchell, Mountain View CA 94041 (US)
(74) Representative: Schnappauf, Georg

(56) References cited:
- WO-A-00/78778
- WO-A-00/78779
- WO-A-2004/011010
- WO-A2-01/62979
- US-A- 6 026 317
- ZHAO G. ET AL: "Comparative profile of vasodilation by CVT-3146, a novel A2a receptor agonist, and adenosine in concious dogs", JOURNAL OF PHARMACOLOGY AND EXPERIMENTAL THERAPEUTICS, vol. 307, 2003, pages 182-189, XP002306850,
- JADBABAIE F. ET AL: "Myocardial perfusion imaging with a novel selective A2a adenosine receptor agonist (CVT.3146): Important differences in radiotracer behaviour", JOURNAL OF THE AMERICAN COLLEGE OF CARDIOLOGY, vol. 41, March 2003 (2003-03), pages 443A-444A, XP008039253,
- GAO Z ET AL: "NOVEL SHORT-ACTING A2A ADENOSINE RECEPTOR AGONISTS FOR CORONARY VASODILATION: INVERSE RELATIONSHIP BETWEEN AFFINITY AND DURATION OFACTION OF A2A AGONISTS", JOURNAL OF PHARMACOLOGY AND EXPERIMENTAL THERAPEUTICS, AMERICAN SOCIETY FOR PHARMACOLOGY AND, US, vol. 298, no. 1, 2001, pages 209-218, XP001021027, ISSN: 0022-3565
- TROCHU J-N ET AL: "SELECTIVE A2A ADENOSINE RECEPTOR AGONIST AS A CORONARY VASODILATOR IN CONSCIOUS DOGS: POTENTIAL FOR USE IN MYOCARDIAL PERFUSION IMAGING", JOURNAL OF CARDIOVASCULAR PHARMACOLOGY, NEW YORK, NY, US, vol. 41, no. 1, 2003, pages 132-139, XP008025427, ISSN: 0160-2446
- CERQUEIRA M. D.: "The future of pharmacologic stress: selective A2a adenosine receptor agonists", AM J. CARDIOL., vol. 94, no. suppl, 2004, pages 33D-42D, XP008039306,

## Description

### BACKGROUND OF THE INVENTION

### (1) Field of the Invention

This invention relates to pharmaceutical compositions for use in myocardial imaging methods that are accomplished by administering doses of one or more adenosine A_{2A} adenosine receptor agonists to a mammal undergoing myocardial imaging.

### (2) Description of the Art

Myocardial perfusion imaging (MPI) is a diagnostic technique useful for the detection and characterization of coronary artery disease. Perfusion imaging uses materials such as radionuclucides to identify areas of insufficient blood flow. In MPI, blood flow is measured at rest, and the result compared with the blood flow measured during exercise on a treadmill (cardiac stress testing), such exertion being necessary to stimulate blood flow. Unfortunately, many patients are unable to exercise at levels necessary to provide sufficient blood flow, due to medical conditions such as peripheral vascular disease, arthritis, and the like.

Therefore, a pharmacological agent that increases cardiac blood flow (CBF) for a short period of time would be of great benefit, particularly one that did not cause peripheral vasodilation. Vasodilators, for example dipyridamole, have been used for this purpose in patients prior to imaging with radionuclide. Dipyridamole is an effective vasodilator, but side effects such as pain and nausea limit the usefulness of treatment with this compound.

Adenosine, a naturally occurring nucleoside, also is useful as a vasodilator. Adenosine exerts its biological effects by interacting with a family of adenosine receptors characterized as subtypes A₁, A_{2A}, A_{2B}, and A₃, Adenoscan^{®}(Fujisawa Healthcare Inc.) is a formulation of a naturally occurring adenosine. Adenoscan^{®} has been marketed as an adjuvant in perfusion studies using radioactive thallium-201. However, its use is limited due to side effects such as flushing, chest discomfort, the urge to breathe deeply, headache, throat, neck, and jaw pain. These adverse effects of adenosine are due to the activation of other adenosine receptor subtypes other than A_{2A}, which mediates the vasodilatory effects of adenosine. Additionally, the short half-life of adenosine necessitates multiple treatments during the procedure, further limiting its use. Adenoscan^{®} is contraindicated in many patients including those with second-or third-degree block, sinus node disease, bronchoconstrictive or bronchospastic lung disease, and in patients with known hypersensitivity to the drug.

WO 00/78779 describes 2-adenosine N-pyrazole compounds and methods for using compounds as A_{2A} receptor agonists to stimulate mammalian coronary vasodilatation for therapeutic purposes and for purposes of imaging the heart. WO 01/62979 discloses a method for identifying and using partial adenosine A_{2A} receptor agonists that are useful as adjuncts in myocardiological perfusion imaging.

Other potent and selective agonists for the A_{2A} adenosine receptor are known. For example, MRE-0470 (Medco) is an adenosine A_{2A} receptor agonist that is a potent and selective derivative of adenosine. WRC-0470 (Medco) is an adenosine A_{2A} agonist used as an adjuvant in imaging. In general, compounds such as these have a high affinity for the A_{2A} receptor, and consequently, a long duration of action, which is undesirable in imaging.

Thus, there is still a need for a method of producing rapid and maximal coronary vasodilation in mammals without causing corresponding peripheral vasodilation, which would be useful for myocardial imaging with radionuclide agents. Preferred compounds would be selective for the A_{2A} adenosine receptor and have a short duration of action (although longer acting than compounds such as adenosine), thus obviating the need for multiple dosing.

### SUMMARY OF THE INVENTION

The present invention provides a pharmaceutical composition comprising
a) the A₂ₐ receptor agonist named (1-{9-[(4S,2R,3R,5R)-3,4-dihydroxy-5-(hydroxymethyl)oxolan-2-yl]-6-aminopurin-2-yl}pyrazol-4-yl)-N-methylcarboxamide (which is also designated as CVT-3146 herein), which has the formula 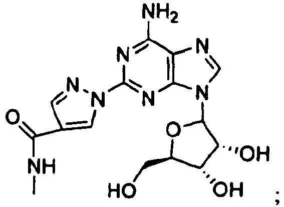
b) at least one liquid carrier selected from the group consisting of water, distilled water, de-ionized water, saline, a buffer, and combinations thereof;
c) propylene glycol in an amount from 5% to 25% (w:v); and
d) EDTA;
wherein the liquid carrier includes a buffer to bring said composition to a pH of from 6 to 8,
for use in a method of myocardial perfusion imaging of a human, said method comprising administering a radionuclide and the A₂ₐ, receptor agonist in an amount of at least 400 µg, wherein the myocardium is examined for areas of insufficient blood flow following administration of the radionuclide and the A₂ₐ receptor agonist.

In one embodiment the pharmaceutical composition is administered by iv bolus. In one embodiment the myocardium examination begins within about 1 minute from the time the A₂ₐ receptor agonist is administered. In one embodiment the administration of the A₂ₐ receptor agonist causes at least a 2.5 fold increase in coronary blood flow. In one embodiment the administration of the A₂ₐ receptor agonist causes at least a 2.5 fold increase in coronary blood flow that is achieved within about I minute from the administration of the A₂ₐ receptor agonist. In one embodiment the radionuclide and the A₂ₐ receptor agonist are administered separately or simultaneously. In one embodiment the administration of the A₂ₐ receptor agonist causes at least a 2.5 fold increase in coronary blood flow for less than about 5 minutes, such as less than about 3 minutes. In one embodiment the A₂ₐ receptor agonist is administered in an amount of up to about 600 µg in a single dose, such as up to about 500 µg in a single dose, or in an amount of about 400 µg in a single dose.

### DESCRIPTION OF THE FIGURES

Figure 1 are intracoronary Doppler flow profiles following administration of 18 µg adenosine IC bolus (top) and 30 µg CVT-3146 IV bolus;
Figure 2 is a plot showing the relationship of the dose of CVT-3146 on coronary peak flow rates;
Figure 3 is a Table that reports the duration of time the coronary flow velocity is greater than or equal to 2.5 times baseline coronary flow velocity for varying doses of CVT-3146 wherein "n" refers to the number of human patients dosed;
Figure 4 is a plot of the time course of the average peak velocity (APV) ratio for human patients receiving 400 µg of CVT-3146 IV bolus;
Figure 5 is a plot of the time course of heart rate for human patients receiving 400 µg of CVT-3146 IV bolus;
Figure 6 is the time course of blood pressure for human patients receiving 400 µg of CVT-3146 IV bolus; and
Figure 7 is an adverse event Table.
Figure 8 is a plot of the change over time of mean CVT-3146 plasma concentration in healthy male volunteers in a supine position. The various curves relate to different amounts of CVT-3146 administered to the patients;
Figures 9 and 10 are plots of the mean change in heart rate of healthy male volunteers either in a standing position or in a supine position over time for various bolus dosing levels of CVT-3146;
Figure 11 is a plot of the maximum change in heart rate in relationship to the total dose of CVT-3146 administered to standing or supine human male patients. In the plot, the term "DBS" refers to the observed data point while "fit" refers to a curve fitted to the observed data points;
Figure 12 is a plot of heart rate - (area under curve) AUC(0-15min) of change from baseline in relationship to the total dose of CVT-3146 administered to standing or supine human subjects;
Figure 13 is a plot of the maximum change from baseline heart rate at maximum plasma concentration of CVT-3146 for patients in a supine position;
Figure 14 is a plot of heart rate - (area under the curve -time v. effect) AUCE (0-15 min) of change from baseline versus plasma AUC(0-15 min) for patients in a supine position;

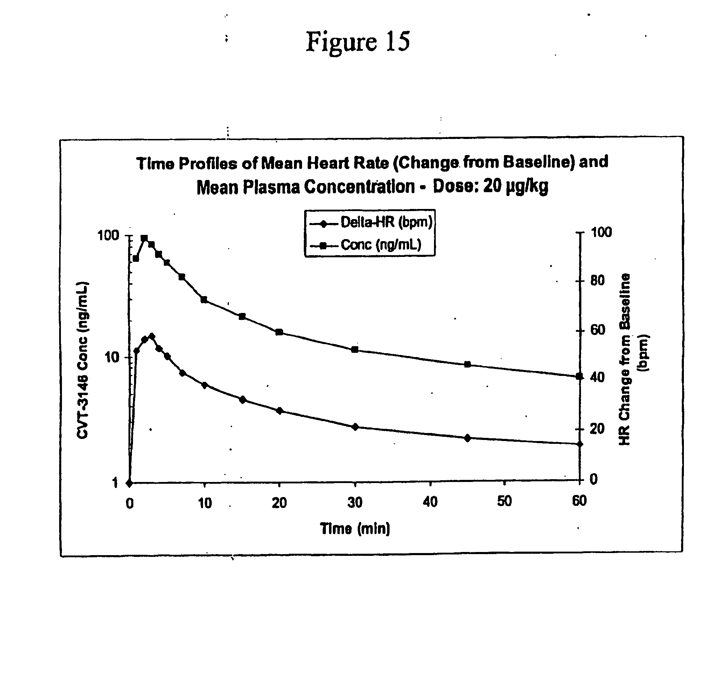

Figure 15 is a plot of the time profiles of mean heart rate change from a baseline versus mean plasma concentration over time for a 20 µg/kg dose of CVT-3146;
Figure 16 is a plot of the average peak to blood flow velocity over time following administration of CVT-3146 measured at the pulmonary artery (PA), the four limb artery (FA), brain arterial vasculature (BA) and in the left circumflex coronary artery (LCS);
Figure 17 is a plot of the percent change in heart rate (HR) and blood pressure (BP) for various doses of CVC-3146; and
Figure 18 is a plot of the change in LBF and RBF blood flow upon administering increasing amounts of ADO or CVT-3146 to awake dogs.

### DESCRIPTION OF THE INVENTION

Potent A_{2A} agonists are useful as adjuncts in cardiac imaging when added either prior to dosing with an imaging agent or simultaneously with an imaging agent. Suitable imaging agents include ²⁰¹Thallium or ^{99m}Technetium-Sestamibi, ^{99mT}teboroxime, and ^{99m}Tc(III).

New and potent A_{2A} agonists that increase CBF but do not significantly increase peripheral blood flow have been identified. The A_{2A} agonists, and especially CVT-3146 and CVT-3033 have a rapid onset and a short duration when administered. An unexpected and newly identified benefit of these new compounds is that they are very useful when administered in a very small quantity in a single bolus intravenous (i.v.) injection. The A_{2A} receptor agonists can be administered in amounts as high as 600 µg or more and still be effective with few if any side-effects. An optimal dose may include as much as 1000 µg or more of a A_{2A} receptor agonist. It is preferred that the A_{2A} receptor agonist is administered in a single bolus injection in an amount selected from about 400 µg, about 500 µg, about 600 µg, and about 700 µg. These amounts are unexpectedly small when compared with adenosine which is typically administered continuously by IV infusion at a rate of about 140 µg/kg/min. Unlike adenosine, the same dosage of A_{2A} receptor agonists, and in particular, CVT-3146 and CVT-3033 can be administered to a human patient regardless of the patient's weight. Thus, the administration of a single uniform amount of a A_{2A} receptor agonists by iv bolus for myocardial imaging is dramatically simpler and less error prone than the time and weight dependent administration of adenosine. The dose of A_{2A} receptor agonist administered to a human patient can, however, be determined by weight. Typically, a weight based dose will be up to about 60 µg/kg and more preferably up to about 30 µg/kg. CVT-3146 in particular is generally well tolerated when administered in an amount up to 10 µg/kg in standing patients and up to 20 µg/kg in supine patients.

A_{2A} agonists may be administered orally, intravenously, through the epidermis or by any other means known in the art for administering therapeutic agents with bolus i.v. administration being preferred. In one embodiment, the bolus dosing occurs in 60 seconds or less. In yet other embodiments, the bolus dosing occurs in about 30 seconds or less, and more preferably in about 20 seconds or less or in about 10 seconds or less.

The A_{2A} agonists are preferably administered in a single dose. The term "single dose" refers generally to a single quickly administered dose of a therapeutic amount of at least one A_{2A} receptor agonist. The term "single dose" does not encompass a dose or doses administered over an extended period of time by, for example continuous i.v. infusion.

The term "pharmaceutical composition" refers to the combination of one or more A_{2A} agonist compounds with at least one liquid carrier that together form a solution or a suspension. Examples of suitable liquid carriers include, but are not limited to water, distilled water, de-ionized water, saline, buffer solutions, normal isotonic saline solution, dextrose in water, and combinations thereof. Such pharmaceutical compositions are generally suitable for injection.
The term "buffer solution" or "buffer" as used herein refers to a solution containing both a weak acid and its conjugate weak base. The buffer solutions are used in pharmaceutical compositions in order to resist pH changes. Non-limiting examples of useful buffer solutions are solutions that comprise sodium bicarbonate and sodium phosphate.

A_{2A} receptor agonists are prepared and then administered, with or without intervening storage, as a pharmaceutical composition. Various properties considered when formulating pharmaceutical compositions include product shelf life, A_{2A} receptor agonist solubility, composition pH, vein irritation, hemolysis, storage conditions (e.g., whether the pharmaceutical composition will be stored at room temperature or some other temperature) and the ability to withstand sterilization procedures.

CVT-3146 has solubility in water of about 50 micrograms/mL. Therefore, CVT-3146 can be dissolved and administered in water so long as the desired weight amount of CVT-3146 can be administered in an acceptable volume. For example, a preferred dose of about 400 micrograms can be administered in 8 mL of water. If this volume is too great for administration purposes, or if the pharmaceutical composition will be stored at other than room temperature (RT), then additional ingredients can be added to the composition to increase the solubility of CVT-3146 in the composition and/or to provide the resulting pharmaceutical composition with other improved properties such as improved stability and storage properties. Pharmaceutical compositions that include CVT-3146 may include up to about 1 milligram/mL of CVT-3146. It is preferred that pharmaceutical compositions including CVT-3146 include from about 50 to about 250 micrograms/mL, and more preferably from about 50 to 150 micrograms/mL of CVT-3146.

The amount of propylene glycol (PG) used in the composition ranges from about 5% to up to 25% by volume with a range of about 8% to about 20% by volume being preferred.

The CVT-3146 composition including PG will have a pH of from 6 to 8 witch pH of about 7 being preferred. Any physiologically acceptable buffer capable of adjusting the composition pH to the desired value can be used. Additional optional ingredients such as dimethylacetamide could be employed in the composition as well.

The pharmaceutical compositions may include one or more anti-oxidants such as butylated hydroxyanisole (BHA).

A first class of compounds that are potent and selective agonists for the A_{2A} adenosine receptor that are useful in the methods described herein are 2-adenosine N-pyrazole compounds having the formula: 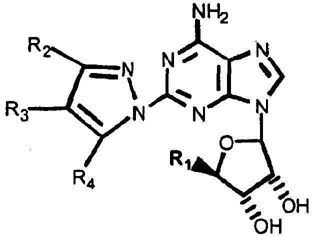
wherein R¹ = CH₂OH, -CONR₅R₆;
R³ is independently selected from the group consisting of C₁₋₁₅ alkyl, halo, NO₂, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂ NR²⁰COR²², NR²⁰CO₂R²², NR²⁰CON(R²⁰)₂, NR²⁰C(NR²⁰)NHR²³, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰, and OCON(R²⁰)₂,-CONR⁷R⁸, C₂₋₁₅ alkenyl, C₂₋₁₅ alkynyl, heterocyclyl, aryl, and heteroaryl, wherein the alkyl, alkenyl, alkynyl, aryl, heterocyclyl and heteroaryl substituents are optionally substituted with from 1 to 3 substituents independently selected from the group consisting of halo, alkyl, NO_{z}, heterocyclyl, aryl, heteroaryl, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂ NR²⁰COR²², NR²⁰CO₂R²², NR²⁰CON(R²⁰)₂, NR²⁰C(NR²⁰)NHR²³, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰, and OCON(R²⁰)₂ and wherein the optional substituted heteroaryl, aryl, and heterocyclyl substituents are optionally substituted with halo, NO₂, alkyl, CF₃, amino, mono- or di- alkylamino, alkyl or aryl or heteroaryl amide, NCOR²², NR²⁰SO₂R²², COR²⁰, CO₂R²⁰, CON(R²⁰ )₂, NR²⁰CON(R²⁰)₂, OC(O)R²⁰, OC(O)N(R²⁰)₂, SR ²⁰, S(O)R²², SO₂R²², SO₂N(R²⁰)₂, CN, or OR²⁰;
R⁵ and R⁶ are each individually selected from H, and C₁-C₁₅ alkyl that is optionally substituted with from 1 to 2 substituents independently selected from the group of halo, NO₂, heterocyclyl, aryl, heteroaryl, CF₃, CN, OR²⁰, SR²⁰, N(R²⁰)₂, S(O)R²¹, SO₂R²², SO₂N(R²⁰)₂, SO₂NR²⁰COR²², SO₂NR²⁰CO₂R²². SO₂NR²⁰CON(R²⁰)₂, N(R²⁰)₂ NR²⁰COR²², NR¹⁰CO₂R²², NR²⁰CON(R²⁰)₂, NR²⁰C(NR²⁰)NHR²³, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰, and OCON(R²⁰)₂ wherein each optional substituted heteroaryl, aryl, and heterocyclyl substituent is optionally substituted with halo, NO₂, alkyl, CF₃, amino, monoalkylamino, dialkylamino, alkylamide, arylamide, heteroarylamide, NCOR²², NR²⁰SO₂R²², COR²⁰, CO₂R ²⁰, CON(R²⁰)₂, NR²⁰CON(R²⁰)_{2,} OC(O)R²⁰, OC(O)N(R²⁰)₂, SR²⁰, S(O)R²², SO₂R²², SO₂N(R²⁰)₂, CN, and OR²⁰;
R⁷ is selected from the group consisting of hydrogen, C₁₋₁₅ alkyl, C₂₋₁₅ alkenyl, C₂₋₁₅ alkynyl, heterocyclyl, aryl and heteroaryl, wherein the alkyl, alkenyl, alkynyl, aryl, heterocyclyl and heteroaryl substituents are optionally substituted with from 1 to 3 substituents independently selected from the group of halo, NO₂, heterocyclyl, aryl, heteroaryl, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂ NR²⁰COR²², NR²⁰CO₂R²², NR²⁰CON(R²⁰)₂, NR²⁰C(NR²⁰)NHR²³, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰ and OCON(R²⁰)₂ and wherein each optional substituted heteroaryl, aryl and heterocyclyl substituent is optionally substituted with halo, NO₂, alkyl, CF₃, amino, mono- or dialkylamino, alkyl or aryl or heteroaryl amide, NCOR²², NR²⁰SO₂*²²,* COR²⁰, CO₂R²⁰, CON(R²⁰)₂, NR²⁰CON(R²⁰)₂, OC(O)R²⁰, OC(O)N(R²⁰)₂, SR²⁰, S(O)R²², SO₂R²², SO₂N(R²⁰)₂, CN, and OR²⁰;
R⁸ is selected from the group consisting of-hydrogen, C₁₋₁₅ alkyl, C₂₋₁₅ alkenyl, C₂₋₁₅ alkynyl, heterocyclyl, aryl, and heteroaryl, wherein the alkyl, alkenyl, alkynyl, aryl, heterocyclyl, and heteroaryl substituents are optionally substituted with from 1 to 3 substituents independently selected from the group consisting of halo, NO₂, heterocyclyl, aryl, heteroaryl, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂ NR²⁰COR²², NR²⁰CO₂R²², NR²⁰CON(R²⁰)₂, NR²⁰C(NR²⁰)NHR²³, COR²⁰, CO₂R²⁰, CON(R²⁰)₂, CONR¹⁰SO₂R22, NR¹⁰SO₂R²², SO₂NR²⁰CO₂R²², OCONR²⁰SO₂R²², OC(O)R²⁰, C(O)OCH₂OC(O)R²⁰, and OCON(R²⁰)₂ and wherein each optional substituted heteroaryl, aryl, and heterocyclyl substituent is optionally substituted with halo, NO₂, alkyl, CF₃, amino, mono- or di- alkylamino, alkyl or aryl or heteroaryl amide, NCOR²², NR²⁰SO₂R²², COR²⁰, CO₂R²⁰, CON(R²⁰)₂, NR²⁰CON(R²⁰)₂, OC(O)R²⁰, OC(O)N(R²⁰)₂, SR²⁰, S(O)R²², SO₂R²², SO₂N(R²⁰)₂, CN, and OR²⁰;
R²⁰ is selected from the group consisting ofH, C₁₋₁₅ alkyl, C₂₋₁₅ alkenyl, C₂₋₁₅ alkynyl, heterocyclyl, aryl, and heteroaryl, wherein the alkyl, alkenyl, alkynyl, heterocyclyl, aryl, and heteroaryl substituents are optionally substituted with from 1 to 3 substituents independently selected from halo, alkyl, mono- or dialkylamino, alkyl or aryl or heteroaryl amide, CN, O-C₁₋₆ alkyl, CF₃, aryl, and heteroaryl;
R²² is selected from the group consisting of C₁₋₁₅ alkyl, C₂₋₁₅ alkenyl, C₂₋₁₅ alkynyl, heterocyclyl, aryl, and heteroaryl, wherein the alkyl, alkenyl, alkynyl, heterocyclyl, aryl, and heteroaryl substituents are optionally substituted with from 1 to 3 substituents independently selected from halo, alkyl, mono- or dialkylamino, alkyl or aryl or heteroaryl amide, CN, O-C₁₋₆ alkyl, CF₃, aryl, and heteroaryl; and wherein R² and R⁴ are selected from the group consisting of H, C₁₋₆ alkyl and aryl, wherein the alkyl and aryl substituents are optionally substituted with halo, CN, CF₃, OR²⁰ and N(R²⁰)₂ with the proviso that when R₂ is not hydrogen then R⁴ is hydrogen, and when R⁴ is not hydrogen then R² is hydrogen.

In an related group of compounds, R³ is selected from the group consisting of C₁₋₁₅ alkyl, halo,CF₃, CN, OR²⁰, SR²⁰, S(O)R²¹, SO₂R²², SO₂N(R²⁰)₂, COR²⁰, CO₂R²⁰, -CONR⁷R⁸, aryl and heteroaryl wherein the alkyl, aryl and heteroaryl substituents are optionally substituted with from 1 to 3 substituents independently selected from the group consisting of halo, aryl, heteroaryl, CF₃, CN, OR²⁰, SR²⁰, S(O)R²². SO₂R²², SO₂N(R²⁰)₂, COR²⁰, CO₂R²⁰ or CON(R²⁰)₂, and each optional heteroaryl and aryl substituent is optionally substituted with halo, alkyl, CF₃ CN, and OR²⁰; R⁵ and R⁶ are independently selected from the group of H and C₁-C₁₅ alkyl including one optional aryl substituent and each optional aryl substituent that is optionally substituted with halo or CF₃; R⁷ is selected from the group consisting of C₁₋₁₅ alkyl, C₂₋₁₅ alkynyl, aryl, and heteroaryl, wherein the alkyl, alkynyl, aryl, and heteroaryl substituents are optionally substituted with from 1 to 3 substituents independently selected from the group consisting of halo, aryl, heteroaryl, CF₃, CN, OR²⁰, and each optional heteroaryl and aryl substituent is optionally substituted with halo, alkyl, CF₃ CN, or OR²⁰; R⁸ is selected from the group consisting ofhydrogen and C₁₋₁₅ alkyl; R²⁰ is selected from the group consisting ofH, C₁₋₄ alkyl and aryl, wherein alkyl and aryl substituents are optionally substituted with one alkyl substituent; and R²² is selected from the group consisting of C₁₋₄ alkyl and aryl which are each optionally substituted with from 1 to 3 alkyl group.

In yet another related class of compounds, R¹ is CH₂OH; R³ is selected from the group consisting of CO₂R²⁰, -CONR⁷R⁸ and aryl where the aryl substituent is optionally substituted with from 1 to 2 substituents independently selected from the group consisting ofhalo, C₁₋₆ alkyl, CF₃ and OR²⁰; R⁷ is selected from the group consisting of hydrogen, C₁₋₈ alkyl and aryl, where the alkyl and aryl substituents are optionally substituted with one substituent selected from the group consisting of halo, aryl, CF₃, CN, OR²⁰ and wherein each optional aryl substituent is optionally substituted with halo, alkyl, CF₃ CN, and OR²⁰ ; R⁸ is selected from the group consisting of hydrogen and C₁₋₃ alkyl; and R²⁰ is selected from hydrogen and C₁₋₄ alkyl.

In a still another related class of compounds, R¹ = CH₂OH; R³ is selected from the group consisting of CO₂R²⁰, -CONR⁷R⁸, and aryl that is optionally substituted with one substituent selected from the group consisting of halo, C₁₋₃ alkyl and OR²⁰; R⁷ is selected from of hydrogen, and C₁₋₃ alkyl; R⁸ is hydrogen; and R²⁰ is selected from hydrogen and C₁₋₄ alkyl. In this preferred embodiment, R² is most preferably selected from -CO₂Et and -CONHEt.

In yet another related class of compounds, R¹ = -CONHEt, R³ is selected from the group consisting of CO₂R²⁰, -CONR⁷R⁸, and aryl in that aryl is optionally substituted with from 1 to 2 substituents independently selected from the group consisting of halo, C₁₋₃ alkyl, CF₃ or OR²⁰; R⁷ is selected from the group consisting of hydrogen, and C₁₋₈ alkyl that is optionally substituted with one substituent selected from the group consisting of halo, CF₃, CN or OR²⁰; R⁸ is selected from the group consisting of hydrogen and C₁₋₃ alkyl; and R²⁰ is selected from the group consisting of hydrogen and C₁₋₄ alkyl. In this more preferred embodiment, R⁸ is preferably hydrogen, R⁷ is preferably selected from the group consisting of hydrogen, and C₁₋₃, and R²⁰ is preferably selected from the group consisting of hydrogen and C₁₋₄ alkyl.

Specific useful compounds are selected from ethyl 1-{9-[(4S,2R,3R,5R)-3,4-dihydroxy-5-(hydroxymethyl)oxolan-2-yl]-6-aminopurin-2-yl}pyrazole-4-carboxylate, (4S,2R,3R,5R)-2-{6-amino-2-[4-(4-chlorophenyl) pyrazolyl]purin-9-yl}-5-(hydroxymethyl)oxolane-3,4-diol, (4S,2R,3R,5R)-2-{6-amino-2-[4-(4-methoxyphenyl)pyrazolyl]purin-9-yl}-5-(hydroxymethyl)oxolane-3,4-diol, (4S,2R,3R,5R)-2-{6-amino-2-[4-(4-methylphenyl)pyrazolyl]purin-9-yl}-5-(hydroxymethyl) oxolane-3,4-diol, (1-{9-[(4S,2R,3R,5R)-3,4-dihydroxy-5-(hydroxymethyl)oxolan-2-yl]-6-aminopurin-2-yl}pyrazol-4-yl)-N-methylcarboxamide, 1-{9-[(4S,2R,3R,5R)-3,4-dihydroxy-5-(hydroxymethyl)oxolan-2-yl]-6-aminopurin-2-yl}pyrazole-4-carboxylic acid, (1-{9-[(4S,2R,3R,5R)-3,4-dihydroxy-5-(hydroxymethyl)oxolan-2-yl]-6-aminopurin-2-yl}pyrazol-4-yl)-N,N-dimethylcarboxamide, (1-{9-[(4S,2R,3R,5R)-3,4-dihydroxy-5-(hydroxymethyl)oxolan-2-yl]-6-aminopurin-2-yl}pyrazol-4-yl)-N-ethylcarboxamide, 1-{9-[(4S,2R,3R,5R)-3,4-dihydroxy-3-(hydroxymethyl)oxolan-2-yl]-6-aminopurin-2-yl}pyrazole-4-carboxamide, 1-{9-[(4S,2R,3R,5R)-3,4-dihydroxy-5-(hydroxymethyl)oxolan-2-yl]-6-aminopurin-2-yl}pyrazol-4-yl}-N-(cyclopentylmethyl)carboxamide, (1-{9-[(4S,2R, 3R,5R)-3,4-dihydroxy-5-(hydroxymethyl)oxolan-2-yl]-6-aminopurin-2-yl} pyrazol-4-yl)-N-[(4-chlorophenyl)methyl]carboxamide, Ethyl 2-[(1-{9-[(4S,2R, 3R,5R)-3,4-dihydroxy-5-(hydroxymethyl)oxolan-2-yl]-6-aminopurin-2-yl}pyrazol-4-yl)carbonylamino]acetate, and mixtures thereof.

A second class of compounds that are potent and selective agonists for the A_{2A} adenosine receptor that are useful in the methods described herein are 2-adenosine C-pyrazole compounds having the following formula: 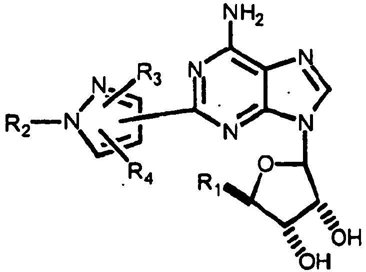
wherein R¹ is -CH₂OH, and -C(=O)NR⁵R⁶;
R² is selected from the group consisting of hydrogen, C₁₋₁₅ alkyl, C₂₋₁₅ alkenyl, C₂₋₁₅ alkynyl, heterocyclyl, aryl, and heteroaryl, wherein the alkyl, alkenyl, alkynyl, aryl, heterocyclyl, and heteroaryl substituents are optionally substituted with from 1 to 3 substituents independently selected from the group consisting of halo, NO₂, heterocyclyl, aryl, heteroaryl, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂ NR²⁰COR²², NR²⁰CO₂R²², NR²⁰CON(R²⁰)₂, NR²⁰C(NR²⁰)NHR²³, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰, and OCON(R²⁰)₂ and wherein each optional heteroaryl, aryl, and heterocyclyl substituent is optionally substituted with halo, NO₂, alkyl, CF₃, amino, mono- or di- alkylamino, alkyl or aryl or heteroaryl amide, NCOR²², NR²⁰SO₂R²², COR²⁰, CO₂R²⁰, CON(R²⁰)₂, NR²⁰CON(R²)₂, OC(O)R²⁰, OC(O)N(R²⁰)₂, SR²⁰, S(O)R²², SO₂R²², SO₂N(R²⁰)₂, CN, or OR²⁰;
R³, R⁴ are individually selected from the group consisting of hydrogen, C₁₋₁₅ alkyl, C₂₋₁₅ alkenyl, C₂₋₁₅ alkynyl, heterocyclyl, aryl, and heteroaryl, halo, NO₂, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂ NR²⁰COR²², NR²⁰CO₂R²², NR²⁰CON(R²⁰)₂, NR²⁰C(NR²⁰)NHR²³, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OCCO)R²⁰, and OCON(R²⁰)₂ wherein the alkyl, alkenyl, alkynyl, aryl, heterocyclyl, and heteroaryl substituents are optionally substituted with from 1 to 3 substituents individually selected from the group consisting of halo, NO₂, heterocyclyl, aryl, heteroaryl, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂ NR²⁰COR²², NR²⁰CO₂R²², NR²⁰CON(R²⁰)₂, NR²⁰C(NR²⁰)NHR²³, COR²⁰, CO₂R²⁰, CON(R²⁰)₂, CONR²⁰SO₂R²², NR²⁰SO₂R²², SO₂NR²⁰CO₂R²², OCONR₂₀SO₂R²², OC(O)R²⁰, C(O)OCH₂OC(O)R²⁰, and OCON(R²⁰)₂ and wherein each optional heteroaryl, aryl, and heterocyclyl substituent is optionally substituted with halo, NO₂, alkyl, CF₃, amino, mono- or di- alkylamino, alkyl or aryl or heteroaryl amide, NCOR²², NR²⁰SO₂R²², COR²⁰, CO₂R²⁰, CON(R²⁰)₂, NR²⁰CON(R²⁰)₂, OC(O)R²⁰, OC(O)N(R²⁰)₂, SR²⁰, S(O)R²², SO₂R²², SO₂N(R²⁰)₂, CN, or OR²⁰;
R⁵ and R⁶ are each individually H, C₁₋₁₅ alkyl with from 1 to 2 substituents independently selected from the group consisting of halo, NO₂, heterocyclyl, aryl, heteroaryl, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂ NR²⁰COR²², NR²⁰CO₂R²², NR²⁰CON(R)₂, NR²⁰C(NR²⁰)NHR²³, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰, and OCON(R²⁰)₂ and wherein each optional heteroaryl, aryl, and heterocyclyl substituent is optionally substituted with halo, NO₂, alkyl, CF₃, amino, mono- or di- alkylamino, alkyl or aryl or heteroaryl amide, NCOR²², NR²⁰SO₂R²², COR²⁰, CO₂R²⁰, CON(R²⁰)₂, NR²⁰CON(R²⁰)₂, OC(O)R²⁰, OC(O)N(R²⁰)₂, SR²⁰, S(O)R²², SO₂R²², SO₂N(R²⁰)₂, CN, or OR²⁰;
R²⁰ is selected from the group consisting of H, C₁₋₁₅ alkyl, C₂₋₁₅ alkenyl, C₂₋₁₅ alkynyl, heterocyclyl, aryl, and heteroaryl, wherein the alkyl, alkenyl, alkynyl, heterocyclyl, aryl, and heteroaryl substituents are optionally substituted with from 1 to 3 substituents independently selected from halo, alkyl, mono- or dialkylamino, alkyl or aryl or heteroaryl amide, CN, O-C₁₋₆ alkyl, CF₃, aryl, and heteroaryl; and
R²² is a member selected from the group consisting of C₁₋₁₅ alkyl,C₂₋₁₅ alkenyl, C₂₋₁₅ alkenyl, heterocyclyl, aryl, and heteroaryl, wherein the alkyl, alkenyl, alkynyl, heterocyclyl, aryl, and heteroaryl substituents are optionally substituted with from 1 to 3 substituents independently selected from halo, alkyl, mono- or dialkylamino, alkyl or aryl or heteroaryl amide, CN, O-C₁₋₆ alkyl, CF₃, and heteroaryl wherein, when R¹ = CH₂OH, R³ is H, R⁴ is H, the pyrazole ring is attached through C⁴, and R² is not H.

When the compound which is selected has one of the following formulas: 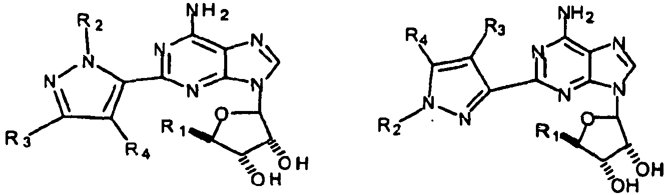 then it is preferred that R¹ is -CH₂OH; R² is selected from the group consisting of hydrogen, C₁₋₈ alkyl wherein the alkyl is optionally substituted with one substituent independently selected from the group consisting of aryl, CF₃, CN, and wherein each optional aryl substituent is optionally substituted with halo, alkyl, CF₃ or CN; and R³ and R⁴ are each independently selected from the group consisting of hydrogen, methyl and more preferably, R³ and R⁴ are each hydrogen.

When the compound has the following formula: 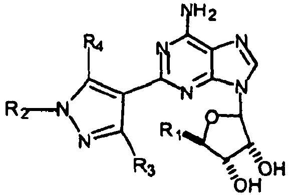 then it is preferred that R¹ is -CH₂OH; R² is selected from the group consisting of hydrogen, and C₁₋₆ alkyl optionally substituted by phenyl. More preferably, R² is selected from benzyl and pentyl; R³ is selected from the group consisting of hydrogen, C₁₋₆ alkyl, aryl, wherein the alkyl, and aryl substituents are optionally substituted with from 1 to 2 substituents independently selected from the group consisting of halo, aryl, CF₃, CN, and wherein each optional aryl substituent is optionally substituted with halo, alkyl, CF₃ or CN; and R⁴ is selected from the group consisting of hydrogen and C₁₋₆ alkyl, and more preferably, R⁴ is selected from hydrogen and methyl.

A more specific class of compounds is selected from the group consisting of (4S,2R,3R,5R)-2-{6-amino-2-[1-benzylpyrazol-4-yl]purin-9-yl}-5-(hydroxymethyl)oxolane-3,4-diol, (4S,2R,3R,5R)-2-[6-amino-2-(1-pentylpyrazol-4-yl)purin-9yl]-5-(hydroxymethyl)oxolane-3,4-diol, (4S,2R,3R,5R)-2-[6-amino-2-(1-methylpyrazol-4-yl)purin-9-yl]-5-(hydroxymethyl)oxolane-3,4-diol, (4S,2R,3R,5R)-2-{6-amino-2-[1-(methylethyl)pyrazol-4-yl]purin-9-yl}-5-(hydroxymethyl)oxolane-3,4-diol, (4S,2R,3R,5R)-2-{6-amino-2-[1-(3-phenylpropyl)pyrazol-4-yl]purin-9-yl}-5-(hydroxymethyl)oxolane-3,4-diol, (4S,2R,3R,5R)-2-{6-amino-2-[1-(4-t-butylbenzyl)pyrazol-4-yl]purin-9-yl}-5-(hydroxymethyl)oxolane-3,4-diol, (4S,2R,3R,5R)-2-(6-amino-2-pyrazol-4-ylpurin-9-yl)-5-(hydroxymethyl)oxolane-3,4-diol, (4S,2R,3R,5R)-2-{6-amino-2-[1-pent-4-enylpyrazol-4-yl]purin-9-yl}-5-(hydroxymethyl)oxolane-3,4-diol, (4S,2R,3R,5R)-2-{6-amino-2-[1-decylpyrazol-4-yl]purin-9-yl}-5-(hydroxymethyl)oxolane-3,4-diol, (4S,2R,3R,5R)-2-{6-amino-2-[1-(cyclohexylmethyl)pyrazol-4-yl]purin-9-yl}-5-(hydroxymethyl)oxolane-3,4-diol, (4S,2R,3R,5R)-2-{6-amino-2-[1-(2-phenylethyl)pyrazol-4-yl]purin-9-yl}-5-(hydroxymethyl)oxolane-3,4-diol, (4S,2R,3R,5R)-2-{6-amino-2-[1-(3-cyclohexylpropyl)pyrazol-4-yl]purin-9-yl]-5-(hydroxymethyl)oxolane-3,4-diol, (4S,2R,3R,5R)-2-{6-amino-2-[1-(2-cyclohexylethyl)pyrazol-4-yl]purin-9-yl}-5-(hydroxymethyl)oxolane-3,4-diol, and combinations thereof.

A very useful and potent and selective agonist for the A_{2A} adenosine receptor is CVT-3146 or (1-{9-[(4S,2R,3R,5R)-3,4-dihydroxy-5-(hydroxymethyl)oxolan-2-yl]-6-aminopurin-2-yl}pyrazol-4-yl)-N-methylcarboxamide which has the formula:

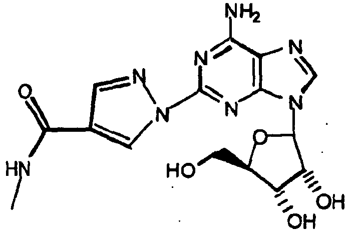

Another preferred compound that is useful as a selective A_{2A}-adenosine receptor agonist with a short duration of action is a compound of the formula: 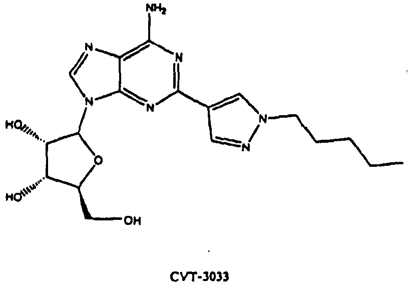 CVT-3033 is particularly useful as an adjuvant in cardiological imaging.

The first and second classes of compounds identified above are described in more detail in U.S. Patent Nos. 6,403,567 and 6,214,807.

The following definitions apply to terms as used herein.

"Halo" or "Halogen" - alone or in combination means all halogens, that is, chloro (Cl), fluoro (F), bromo (Br), iodo (I).

"Hydroxyl" refers to the group -OH.

"Thiol" or "mercapto" refers to the group -SH.

"Alkyl" - alone or in combination means an alkane-derived radical containing from 1 to 20, preferably 1 to 15, carbon atoms (unless specifically defined). It is a straight chain alkyl, branched alkyl or cycloalkyl. Preferably, straight or branched alkyl groups containing from 1-15, more preferably 1 to 8, even more preferably 1-6, yet more preferably 1-4 and most preferably 1-2, carbon atoms, such as methyl, ethyl, propyl, isopropyl, butyl, t-butyl and the like. The term "lower alkyl" is used herein to describe the straight chain alkyl groups described immediately above. Preferably, cycloalkyl groups are monocyclic, bicyclic or tricyclic ring systems of 3-8, more preferably 3-6, ring members per ring, such as cyclopropyl, cyclopentyl, cyclohexyl, adamantyl and the like. Alkyl also includes a straight chain or branched alkyl group that contains or is interrupted by a cycloalkyl portion. The straight chain or branched alkyl group is attached at any available point to produce a stable compound. Examples of this include, but are not limited to, 4-(isopropyl)-cyclohexylethyl or 2-methyl-cyclopropylpentyl. A substituted alkyl is a straight chain alkyl, branched alkyl, or cycloalkyl group defined previously, independently substituted with 1 to 3 groups or substituents of halo, hydroxy, alkoxy, alkylthio, alkylsulfinyl, alkylsulfonyl, acyloxy, aryloxy, heteroaryloxy, amino optionally mono- or di-substituted with alkyl, aryl or heteroaryl groups, amidino, urea optionally substituted with alkyl, aryl, heteroaryl or heterocyclyl groups, aminosulfonyl optionally N-mono- or N,N-di-substituted with alkyl, aryl or heteroaryl groups, alkylsulfonylamino, arylsulfonylamino, heteroarylsulfonylamino, alkylcarbonylamino, arylcarbonylamino, heteroarylcarbonylamino, or the like.

"Alkenyl" - alone or in combination means a straight, branched, or cyclic hydrocarbon containing 2-20, preferably 2-17, more preferably 2-10, even more preferably2-8, most preferably 2-4, carbon atoms and at least one, preferably 1-3, more preferably 1-2, most preferably one, carbon to carbon double bond. In the case of a cycloalkyl group, conjugation of more than one carbon to carbon double bond is not such as to confer aromaticity to the ring. Carbon to carbon double bonds may be either contained within a cycloalkyl portion, with the exception of cyclopropyl, or within a straight chain or branched portion. Examples of alkenyl groups include ethenyl, propenyl, isopropenyl, butenyl, cyclohexenyl, cyclohexenylalkyl and the like. A substituted alkenyl is the straight chain alkenyl, branched alkenyl or cycloalkenyl group defined previously, independently substituted with 1 to 3 groups or substituents of halo, hydroxy, alkoxy, alkylthio, alkylsulfinyl, alkylsulfonyl, acyloxy, aryloxy, heteroaryloxy, amino optionally mono- or di-substituted with alkyl, aryl or heteroaryl groups, amidino, urea optionally substituted with alkyl, aryl, heteroaryl or heterocyclyl groups, aminosulfonyl optionally N-mono- or N,N-di-substituted with alkyl, aryl or heteroaryl groups, alkylsulfonylamino, arylsulfonylamino, heteroarylsulfonylamino, alkylcarbonylamino, arylcarbonylamino, heteroarylcarbonylamino, carboxy, alkoxycarbonyl, aryloxycarbonyl, heteroaryloxycarbonyl, or the like attached at any available point to produce a stable compound.

"Alkynyl" - alone or in combination means a straight or branched hydrocarbon containing 2-20, preferably 2-17, more preferably 2-10, even more preferably 2-8, most preferably 2-4, carbon atoms containing at least one, preferably one, carbon to carbon triple bond. Examples of alkynyl groups include ethynyl, propynyl, butynyl and the like. A substituted alkynyl refers to the straight chain alkynyl or branched alkenyl defined previously, independently substituted with 1 to 3 groups or substituents of halo, hydroxy, alkoxy, alkylthio, alkylsulfinyl, alkylsulfonyl, acyloxy, aryloxy, heteroaryloxy, amino optionally mono- or di-substituted with alkyl, aryl or heteroaryl groups, amidino, urea optionally substituted with alkyl, aryl, heteroaryl or heterocyclyl groups, aminosulfonyl optionally N-mono- or N,N-di-substituted with alkyl, aryl or heteroaryl groups, alkylsulfonylamino, arylsulfonylamino, heteroarylsulfonylamino, alkylcarbonylamino, arylcarbonylamino, heteroarylcarbonylamino, or the like attached at any available point to produce a stable compound.

"Alkyl alkenyl" refers to the group -R-CR'=CR'"R"", where R is lower alkyl, or substituted lower alkyl, R', R"', R"" may independently be hydrogen, halogen, lower alkyl, substituted lower alkyl, acyl, aryl, substituted aryl, hetaryl, or substituted hetaryl as defined below.

"Alkyl alkynyl" refers to the group -RC≡CR' where R is lower alkyl or substituted lower alkyl, R' is hydrogen, lower alkyl, substituted lower alkyl, acyl, aryl, substituted aryl, hetaryl, or substituted hetaryl as defined below.

"Alkoxy" denotes the group -OR, where R is lower alkyl, substituted lower alkyl, acyl, aryl, substituted aryl, aralkyl, substituted aralkyl, heteroalkyl, heteroarylalkyl, cycloalkyl, substituted cycloalkyl, cycloheteroalkyl, or substituted cycloheteroalkyl as defined.

"Alkylthio" denotes the group -SR, -S(O)ₙ₋₁₋₂-R, where R is lower alkyl, substituted lower alkyl, aryl, substituted aryl, aralkyl or substituted aralkyl as defined herein.

"Acyl" denotes the group -C(O)R, where R is hydrogen, lower alkyl substituted lower alkyl, aryl, substituted aryl and the like as defined herein.

"Aryloxy" denotes the group -OAr, where Ar is an aryl, substituted aryl, heteroaryl, or substituted heteroaryl group as defined herein.

"Amino" denotes the group NRR', where R and R' may independently by hydrogen, lower alkyl, substituted lower alkyl, aryl, substituted aryl, hetaryl, or substituted hetaryl as defined herein or acyl.

"Amido" denotes the group -C(O)NRR', where R and R' may independently by hydrogen, lower alkyl, substituted lower alkyl, aryl, substituted aryl, hetaryl, substituted hetaryl as defined herein.

"Carboxyl" denotes the group -C(O)OR, where R is hydrogen, lower alkyl, substituted lower alkyl, aryl, substituted aryl, hetaryl, and substituted hetaryl as defined herein.

"Aryl" - alone or in combination means phenyl or naphthyl optionally carbocyclic fused with a cycloalkyl of preferably 5-7, more preferably 5-6, ring members and/or optionally substituted with 1 to 3 groups or substituents of halo, hydroxy, alkoxy, alkylthio, alkylsulfinyl, alkylsulfonyl, acyloxy, aryloxy, heteroaryloxy, amino optionally mono- or di-substituted with alkyl, aryl or heteroaryl groups, amidino, urea optionally substituted with alkyl, aryl, heteroaryl or heterocyclyl groups, aminosulfonyl optionally N-mono- or N,N-di-substituted with alkyl, aryl or heteroaryl groups, alkylsulfonylamino, arylsulfonylamino, heteroarylsulfonylamino, alkylcarbonylamino, arylcarbonylamino, heteroarylcarbonylamino, or the like.

"Substituted aryl" refers to aryl optionally substituted with one or more functional groups, *e.g*., halogen, lower alkyl, lower alkoxy, alkylthio, acetylene, amino, amido, carboxyl, hydroxyl, aryl, aryloxy, heterocycle, hetaryl, substituted hetaryl, nitro, cyano, thiol, sulfamido and the like.

"Heterocycle" refers to a saturated, unsaturated, or aromatic carbocyclic group having a single ring (*e.g*., morpholino, pyridyl or furyl) or multiple condensed rings (*e.g*., naphthpyridyl, quinoxalyl, quinolinyl, indolizinyl or benzo[b]thienyl) and having at least one hetero atom, such as N, O or S, within the ring, which can optionally be unsubstituted or substituted with, *e.g*., halogen, lower alkyl, lower alkoxy, alkylthio, acetylene, amino, amido, carboxyl, hydroxyl, aryl, aryloxy, heterocycle, hetaryl, substituted hetaryl, nitro, cyano, thiol, sulfamido and the like.

"Heteroaryl" - alone or in combination means a monocyclic aromatic ring structure containing 5 or 6 ring atoms, or a bicyclic aromatic group having 8 to 10 atoms, containing one or more, preferably 1-4, more preferably 1-3, even more preferably 1-2, heteroatoms independently selected from the group O, S, and N, and optionally substituted with 1 to 3 groups or substituents of halo, hydroxy, alkoxy, alkylthio, alkylsulfinyl, alkylsulfonyl, acyloxy, aryloxy, heteroaryloxy, amino optionally mono- or di-substituted with alkyl, aryl or heteroaryl groups, amidino, urea optionally substituted with alkyl, aryl, heteroaryl or heterocyclyl groups, aminosulfonyl optionally N-mono- or N,N-di-substituted with alkyl, aryl or heteroaryl groups, alkylsulfonylamino, arylsulfonylamino, heteroarylsulfonylamino, alkylcarbonylarnino, arylcarbonylamino, heteroarylcarbonylamino, or the like. Heteroaryl is also intended to include oxidized S or N, such as sulfinyl, sulfonyl and N-oxide of a tertiary ring nitrogen. A carbon or nitrogen atom is the point of attachment of the heteroaryl ring structure such that a stable aromatic ring is retained. Examples of heteroaryl groups are pyridinyl, pyridazinyl, pyrazinyl, quinazolinyl, purinyl, indolyl, quinolinyl, pyrimidinyl, pyrrolyl, oxazolyl, thiazolyl, thienyl, isoxazolyl, oxathiadiazolyl, isothiazolyl, tetrazolyl, imidazolyl, triazinyl, furanyl, benzofuryl, indolyl and the like. A substituted heteroaryl contains a substituent attached at an available carbon or nitrogen to produce a stable compound.

"Heterocyclyl" - alone or in combination means a non-aromatic cycloalkyl group having from 5 to 10 atoms in which from 1 to 3 carbon atoms in the ring are replaced by heteroatoms of O, S or N, and are optionally benzo fused or fused heteroaryl of 5-6 ring members and/or are optionally substituted as in the case of cycloalkyl. Heterocycyl is also intended to include oxidized S or N, such as sulfinyl, sulfonyl and N-oxide of a tertiary ring nitrogen. The point of attachment is at a carbon or nitrogen atom. Examples of heterocyclyl groups are tetrahydrofuranyl, dihydropyridinyl, piperidinyl, pyrrolidinyl, piperazinyl, dihydrobenzofuryl, dihydroindolyl, and the like. A substituted hetercyclyl contains a substituent nitrogen attached at an available carbon or nitrogen to produce a stable compound.

"Substituted heteroaryl" refers to a heterocycle optionally mono or poly substituted with one or more functional groups, *e.g*., halogen, lower alkyl, lower alkoxy, alkylthio, acetylene, amino, amido, carboxyl, hydroxyl, aryl, aryloxy, heterocycle, substituted heterocycle, hetaryl, substituted hetaryl, nitro, cyano, thiol, sulfamido and the like.

"Aralkyl" refers to the group -R-Ar where Ar is an aryl group and R is lower alkyl or substituted lower alkyl group. Aryl groups can optionally be unsubstituted or substituted with, *e.g*., halogen, lower alkyl, alkoxy, alkylthio, acetylene, amino, amido, carboxyl, hydroxyl, aryl, aryloxy, heterocycle, substituted heterocycle, hetaryl, substituted hetaryl, nitro, cyano, thiol, sulfamido and the like.

"Heteroalkyl" refers to the group -R-Het where Het is a heterocycle group and R is a lower alkyl group. Heteroalkyl groups can optionally be unsubstituted or substituted with *e.g*., halogen, lower alkyl, lower alkoxy, alkylthio, acetylene, amino, amido, carboxyl, aryl, aryloxy, heterocycle, substituted heterocycle, hetaryl, substituted hetaryl, nitro, cyano, thiol, sulfamido and the like.

"Heteroarylalkyl" refers to the group -R-HetAr where HetAr is an heteroaryl group and R lower alkyl or substituted lower alkyl. Heteroarylalkyl groups can optionally be unsubstituted or substituted with, *e.g*., halogen, lower alkyl, substituted lower alkyl, alkoxy, alkylthio, acetylene, aryl, aryloxy, heterocycle, substituted heterocycle, hetaryl, substituted hetaryl, nitro, cyano, thiol, sulfamido and the like.

"Cycloalkyl" refers to a divalent cyclic or polycyclic alkyl group containing 3 to 15 carbon atoms.

"Substituted cycloalkyl" refers to a cycloalkyl group comprising one or more substituents with, *e.g*., halogen, lower alkyl, substituted lower alkyl, alkoxy, alkylthio, acetylene, aryl, aryloxy, heterocycle, substituted heterocycle, hetaryl, substituted hetaryl, nitro, cyano, thiol, sulfamido and the like.

"Cycloheteroalkyl" refers to a cycloalkyl group wherein one or more of the ring carbon atoms is replaced with a heteroatom (*e.g.,* N, O, S or P).

Substituted cycloheteroalkyl" refers to a cycloheteroalkyl group as herein defined which contains one or more substituents, such as halogen, lower alkyl, lower alkoxy, alkylthio, acetylene, amino, amido, carboxyl, hydroxyl, aryl, aryloxy, heterocycle, substituted heterocycle, hetaryl, substituted hetaryl, nitro, cyano, thiol, sulfamido and the like.

"Alkyl cycloalkyl" denotes the group -R-cycloalkyl where cycloalkyl is a cycloalkyl group and R is a lower alkyl or substituted lower alkyl. Cycloalkyl groups can optionally be unsubstituted or substituted with *e.g*. halogen, lower alkyl, lower alkoxy, alkylthio, acetylene, amino, amido, carboxyl, hydroxyl, aryl, aryloxy, heterocycle, substituted heterocycle, hetaryl, substituted hetaryl, nitro, cyano, thiol, sulfamido and the like.

"Alkyl cycloheteroalkyl" denotes the group -R-cycloheteroalkyl where R is a lower alkyl or substituted lower alkyl. Cycloheteroalkyl groups can optionally be unsubstituted or substituted with *e.g*. halogen, lower alkyl, lower alkoxy, alkylthio, amino, amido, carboxyl, acetylene, hydroxyl, aryl, aryloxy, heterocycle, substituted heterocycle, hetaryl, substituted hetaryl, nitro, cyano, thiol, sulfamido and the like.

The first class of compounds identified above can be prepared as outlined in Schemes 1-4.
Compounds having the general formula IV can be prepared as shown in Scheme 1. 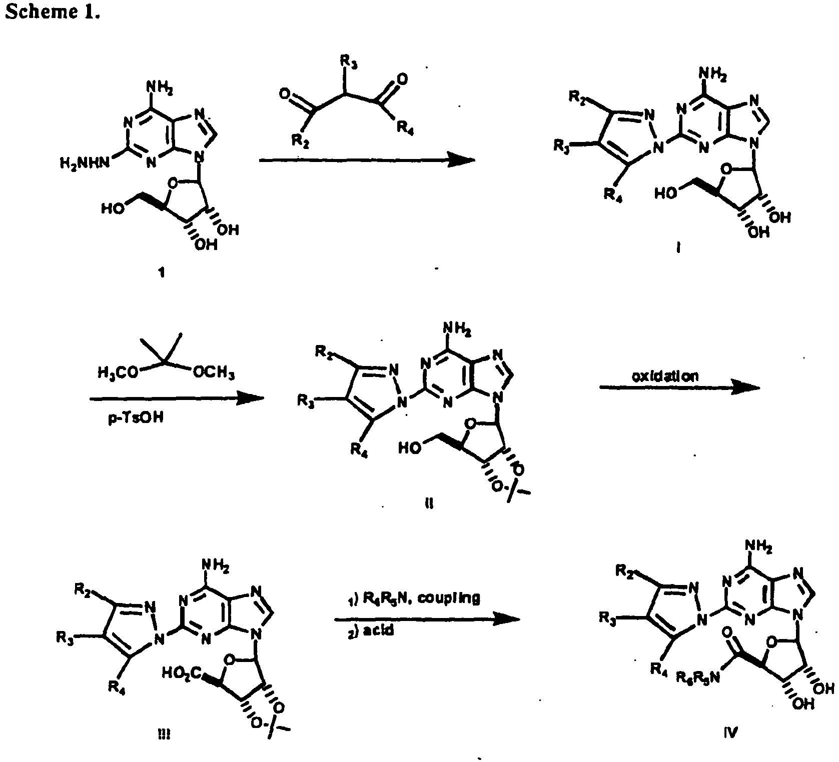 Compound I can be prepared by reacting compound 1 with appropriately substituted 1,3-dicarbonyl in a mixture of AcOH and MeOH at 80°C (Holzer et al., J. Heterocycl. Chem. (1993) 30, 865). Compound II, which can be obtained by reacting compound I with 2,2-dimethoxypropane in the presence of an acid, can be oxidized to the carboxylic acid III, based on structurally similar compounds using potassium permanganate or pyridinium chlorochromate (M. Hudlicky, (1990) Oxidations in Organic Chemistry, ACS Monographs, American Chemical Society, Washington D. C.). Reaction of a primary or secondary amine having the formula HNR⁶R⁷, and compound III using DCC (M. Fujino et al., Chem. Pharm. Bull. (1974), 22, 1857), PyBOP (J. Martinez et al., J. Med. Chem. (1988) 28, 1874) or PyBrop (J. Caste et al., Tetrahedron, (1991), 32, 1967) coupling conditions can afford compound IV.

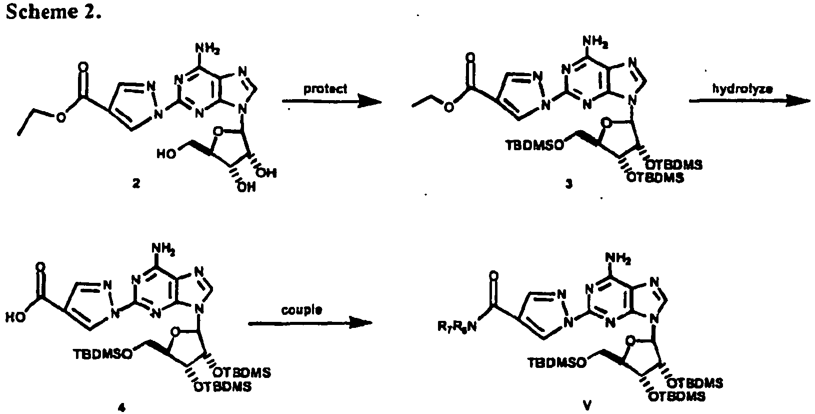

Compound V can be prepared as shown in Scheme 2. The Tri TBDMS derivative 4 can be obtained by treating compound 2 with TBDMSCl and imidazole in DMF followed by hydrolysis of the ethyl ester using NaOH. Reaction of a primary or secondary amine with the formula HNR⁶R⁷, and compound 4 using DCC (M. Fujino et al., Chem. Pharm. Bull. (1974), 22, 1857), PyBOP (J. Martinez et al., J. Med. Chem. (1988) 28, 1874) or PyBrop (J. Caste et al., Tetrahedron, (1991), 32, 1967) coupling conditions can afford compound V.

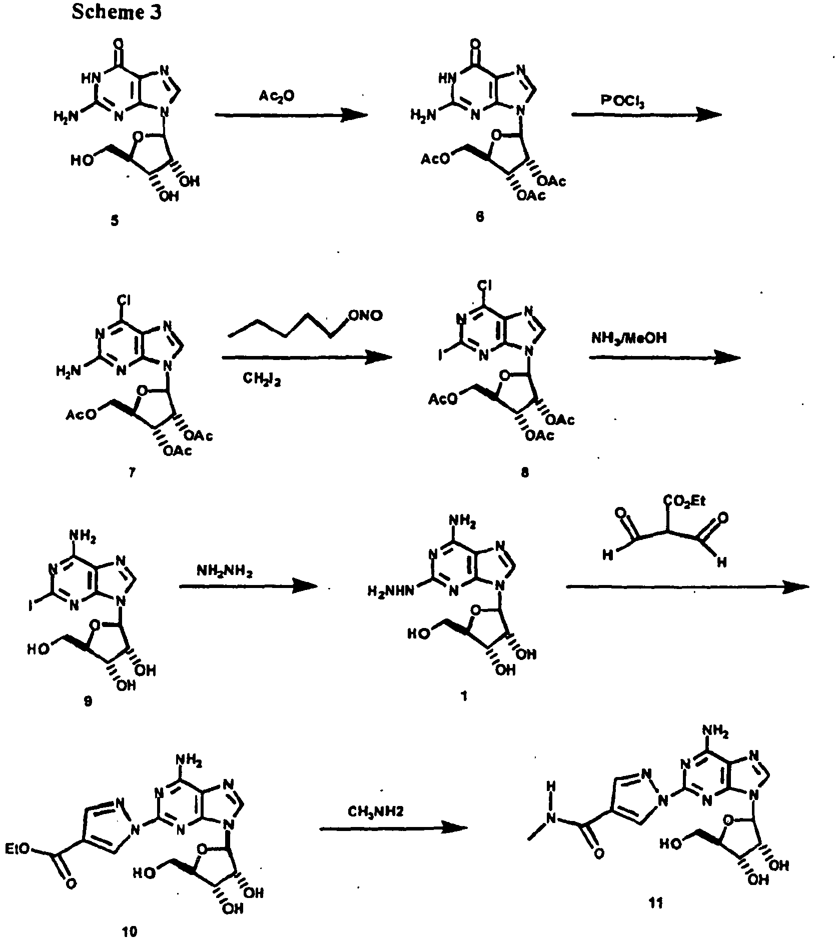

A specific synthesis of Compound 11 is illustrated in Scheme 3. Commercially available guanosine 5 was converted to the triacetate 6 as previously described (M. J. Robins and B. Uznanski, Can. J. Chem. (1981), 59,2601-2607). Compound 7, prepared by following the literature procedure of Cerster et al. (J. F. Cerster, A. F. Lewis, and R.K. Robins, Org. Synthesis, 242-243), was converted to compound 9 in two steps as previously described (V. Nair et al., J. Org. Chem., (1988), 53, 3051-3057). Compound 1 was obtained by reacting hydrazine hydrate with compound 9 in ethanol at 80°C. Condensation of compound 1 with ethoxycarbonylmalondialdehyde in a mixture of AcOH and MeOH at 80°C produced compound 10. Heating compound 10 in excess methylamine afforded compound 11.

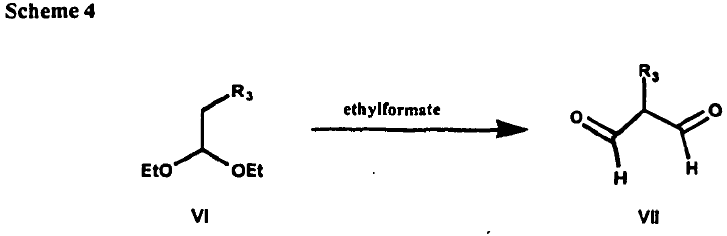

The synthesis of 1,3-dialdehyde VII is described in Scheme 4. Reaction of 3,3-diethoxypropionate or 3,3-diethoxypropionitrile or 1,1-diethoxy-2-nitroethane VI (R₃ = CO₂R, CN or NO₂) with ethyl or methyl formate in the presence of NaH can afford the dialdehyde VII (Y. Yamamoto et al., J. Org. Chem. (1989) 54, 4734).

The second class of compounds described above may be prepared as outlined in schemes 5-9. Compounds having the general formula II: 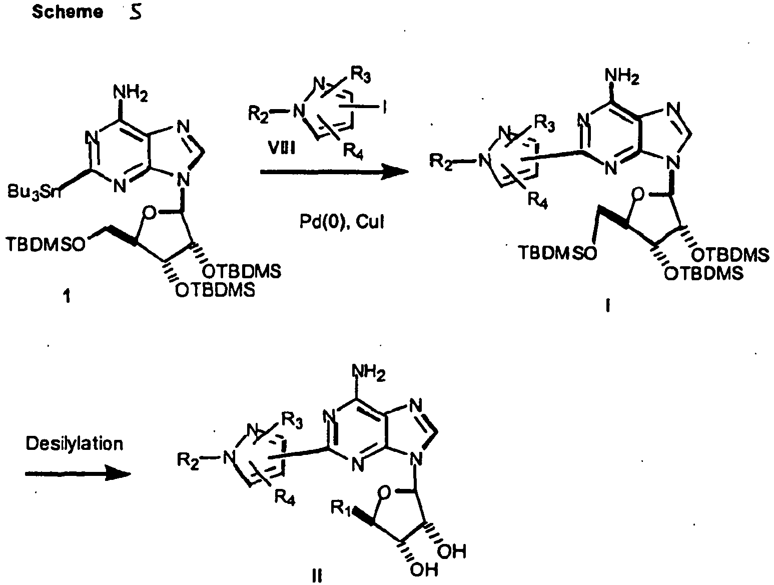 were prepared by the palladium mediated coupling of compound 1 with halo-pyrazoles represented by the formula VIII (scheme 8) in the presence or absence of copper salts (K. Kato et. al., J. Org. Chem. 1997, 62, 6833-6841; Palladium Reagents and Catalysts-Innovations in Organic Synthesis, Tsuji, John Wiley and Sons, 1995) followed by de-protection with either TBAF or NH₄F(Markiewicz et. al., Tetrahedron Lett. (1988), 29, 1561). The preparation of compound 1 has been previously described (K. Kato et. al., J. Org. Chem. 1997, 62, 6833-6841) and is outlined in scheme 11.

Compounds with general formula VI can be prepared as shown in Scheme 6. Compound III, which can be obtained by reacting II with 2,2-dimethoxypropane in presence of an 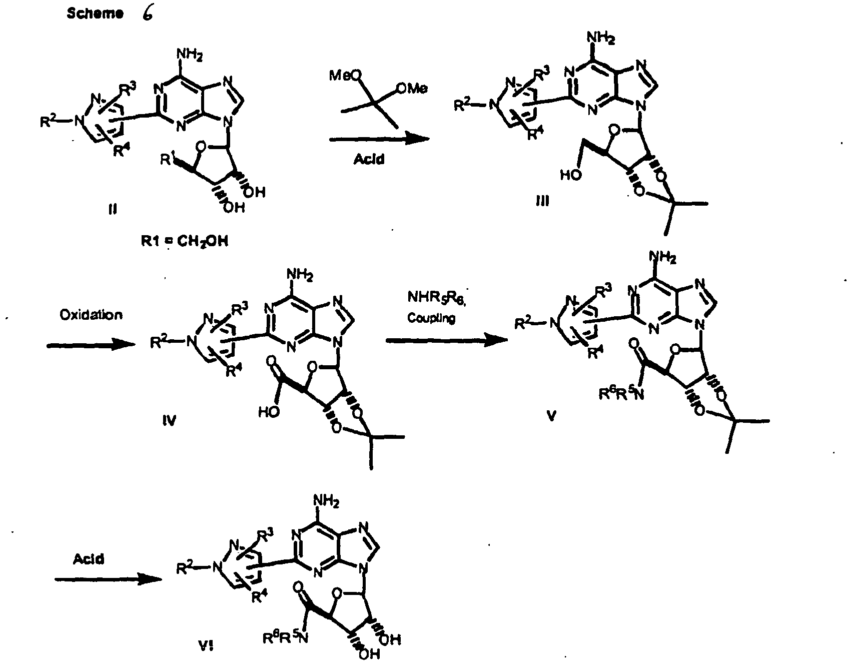 acid, can be oxidized to the carboxylic acid IV, based on structurally similar compounds, using potassium permanganate or pyridinium chlorochromate etc. (Jones et al., J. Am. Chem. Soc. (1949), 71, 3994; Hudlicky, Oxidations in organic chemistry, American Chemical Society, Washington D. C., 1990). Reaction of primary or secondary amine of the formula NHR⁵R⁶, and compound IV using DCC (Fujino et al., Chem. Pharm. Bull. (1974), 22, 1857), PyBOP (J. Martinez et al., J. Med. Chem. (1988), 28, 1967) or PyBrop (J. Caste et al. Tetrahedron, (1991), 32, 1967) coupling conditions can afford compound V. Deprotection of compound V can be performed by heating with 80% aq. acetic acid (T. W. Green and P. G. M. Wuts, (1991), Protective Groups in Organic Synthesis, A, Wiley-Interscience publication) or with anhydrous HCl (4N) to obtain compound of the general formula VI.

Alternatively, compounds with the general formula II can also be prepared by Suzuki type coupling as shown in scheme 7. 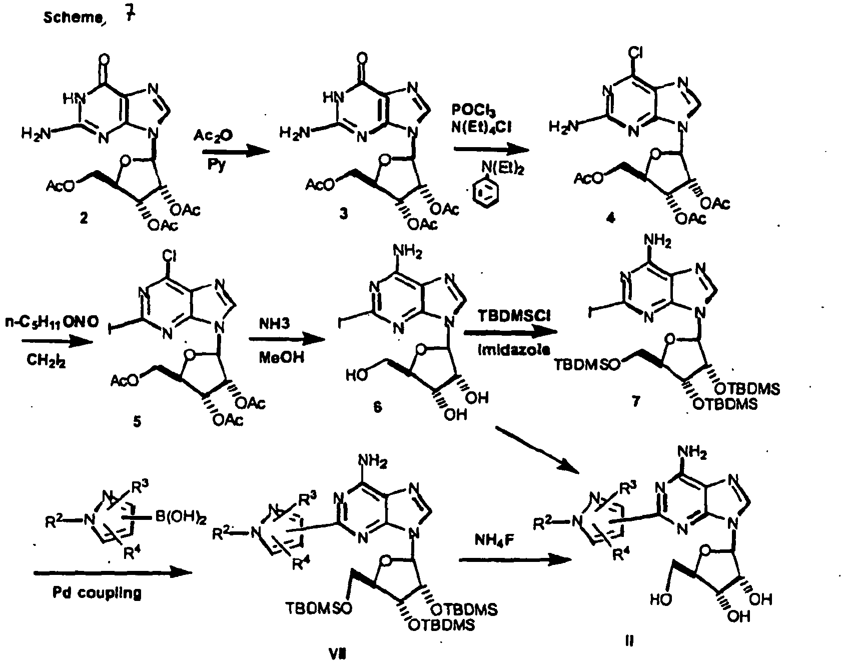 2-Iodoadenosine 6 can be prepared in four steps from guanosine 2 following literature procedures (M. J. Robins et al., Can. J. Chem. (1981), 59, 2601-2607; J. F. Cerster et al., Org. Synthesis, ---242-243; V. Nair et al., J. Org. Chem., (1988), 53, 3051-3057). Palladium mediated Suzuki coupling of 6 with appropriately substituted pyrazole-boronic acids XVII in presence of a base can provide final compounds with general formula II (A.Suzuki, Acc. Chem. Res. (1982), 15, 178). If necessary, 2', 3', 5' hydroxyls on 6 can be protected as TBDMS ethers prior to Suzuki coupling.

Compounds with the general formula VIII can be either commercially available or prepared following the steps shown in scheme 8. 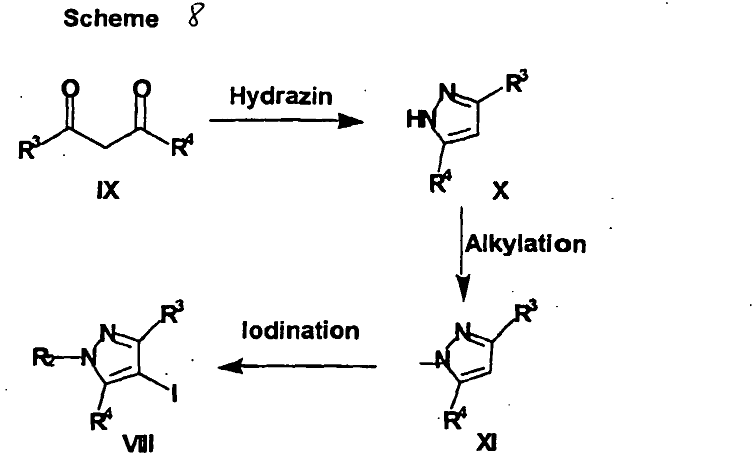 Condensation of 1,3-diketo compounds of the formula IX with hydrazine in an appropriate solvent can give pyrazoles with the general formula X (R.H.Wiley et al., Org. Synthesis, Coll. Vol IV (1963), 351). These pyrazoles can be N-alkylated with various alkyl halides to give compounds of the formula XI which on iodination give 4-iodo derivatives with the general formula VIII (R. Huttel et al., Justus Liebigs Ann.Chem. (1955), 593, 200).

5-Todopyrazoles with the general formula XV can be prepared following the steps outlined in the scheme 9. 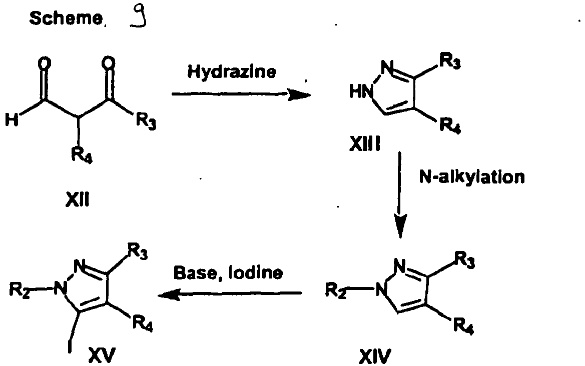 Condensation of 1,3-diketo compounds of the formula XII with hydrazine in an appropriate solvent can give pyrazoles with the general formula XIII. These pyrazoles can be N-alkylated with various alkyl halides to give compounds of the formula XIV. Abstraction of 5-H with a strong base followed by quenching with iodine can provide 5-iodo derivatives with general formula XV (F. Effenberger et. al., J. Org. Chem. (1984), 49, 4687).

4- or 5- iodopyrazoles can be transformed into corresponding boronic acids as shown in the scheme 10. Transmetallation with n-buLi followed by treatment with trimethylborate can 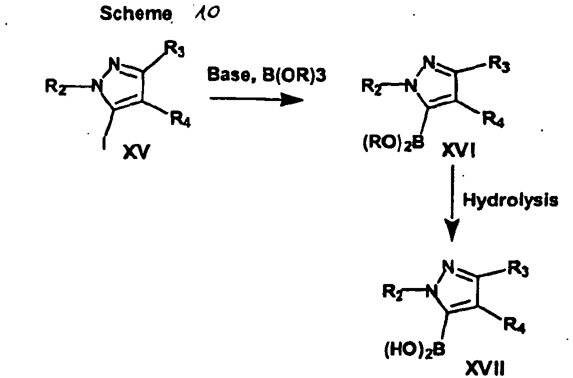 give compounds with the general formula XVI which on hydrolysis can provide boronic acids with the general formula XVII (F. C. Fischer et al., RECUEIL (1965), 84, 439).

2-Stannyladenosine 1 was prepared in three steps from the commercially available 6-chloropurine riboside following literature procedure (K. Kato et al., J. Org. Chem. (1997), 62, 6833-6841). Tri TBDMS derivative was obtained by treating 8 with TBDMSCI and imidazole in DMF. Lithiation with LTMP followed by quenching with tri n-butyltin chloride gave exclusively 2-stannyl derivative 10. Ammonolysis in 2-propanol gave 2-stannyladenosine 1. Stille coupling of 1 with 1-benzyl-4-iodopyrazole in presence of Pd (PPh₃)₄ and CuI resulted in 11 (K. Kato et al., J. Org. Chem. (1997), 62, 6833-6841). Deprotection of silyl groups on 2',3' and 5' 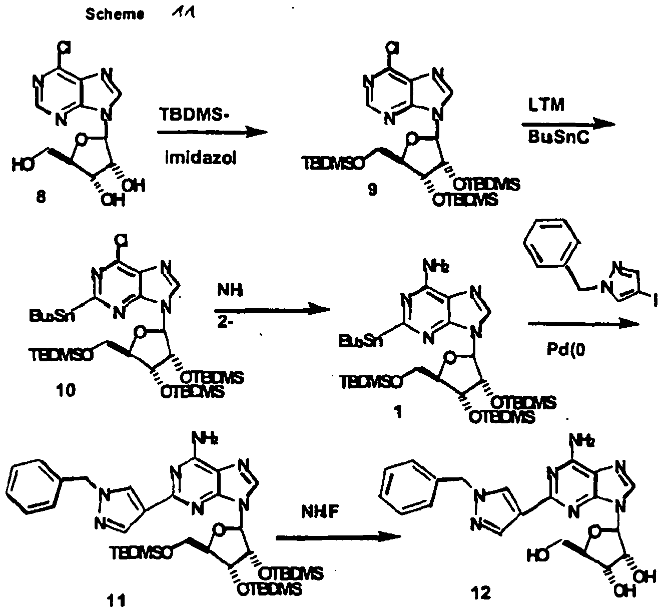 hydroxyls with 0.5 M ammonium fluoride in methanol gave 12 in good yield (Scheme 11). The methods used to prepare the compounds are not limited to those described above. Additional methods can be found in the following sources (J. March, Advanced Organic Chemistry; Reaction Mechanisms and Studies (1992), A Wiley Interscience Publications; and J. Tsuji, Palladium reagents and catalysts-Innovations in organic synthesis, John Wiley and Sons, 1995).

If the final compound contains a basic group, an acid addition salt may be prepared. Acid addition salts of the compounds are prepared in a standard manner in a suitable solvent from the parent compound and an excess of acid, such as hydrochloric, hydrobromic, sulfuric, phosphoric, acetic, maleic, succinic, or methane sulfonic. The hydrochloric salt form is especially useful. If the final compound contains an acidic group, cationic salts may be prepared. Typically the parent compound is treated with an excess of an alkaline reagent, such as hydroxide, carbonate or alkoxide, containing the appropriate cation. Cations such as Na⁺, K⁺, Ca⁺² and NH₄⁺ are examples of cations present in pharmaceutically acceptable salts. Certain of the compounds form inner salts or zwitterions which may also be acceptable. '

### EXAMPLES

Three different aqueous pharmaceutical CVT-3146 formulations were used in the Examples below. Those examples which are not encompassed by the claims are given for comparative purposes only.

Examples 1-7 employed aqueous pharmaceutical compositions (a) and (b) below. Pharmaceutical compositions (a) and (b) were aseptically filled into 10 mL type I glass vials.
(a) An aqueous pharmaceutical composition consisting of 200 micrograms/mL of CVT-3146 in 0.5 (w:v) methylboronic acid buffered with sodium bicarbonate to a pH of 9.3.
(b) An aqueous pharmaceutical composition including 200 micrograms/mL of CVT-3146, 0.1 % (w:v) methylboronic acid, 50 mM sodium bicarbonate buffer adjusted to pH 9.3 with the addition of 0.55 % (w:v) NaCl to make an isotonic pharmaceutical composition.

Example 8 employed a pharmaceutical composition consisting of an aqueous composition of 100 micrograms/mL CVT-3146 in 15% (w:v) propylene glycol and 100 mM phosphate buffer at pH 7 with 0.1 % EDTA. The formulation was stored in type I glass vials at 5 mL per vial.

### EXAMPLE 1

BACKGROUND: CVT-3146 (CVT), with an initial half-life of 3 minutes with a rapid onset and offset of action, is > 100 -fold more potent than adenosine (Ado) in increasing coronary blood flow velocity (CBFv) in awake dogs. The purpose of this open label study was to determine the magnitude and duration of effect of CVT-3146 (10-500 µg) on CBFv in humans.

METHODS: Patients undergoing a clinically indicated coronary catheterization with no more than a 70% stenosis in any coronary artery and no more than a 50% stenosis of the study vessel had CBFv determined by Doppler flow wire. Study subject were selected after measuring baseline and peak CBFv after an intracoronary (IC) injection of 18 µg of Ado. Twenty-three patients, who were identified as meeting the study criteria of having a peak to baseline CBFv ratio of ≥ 2.5 in response to Adenosine, received a rapid (≤ 10 sec) peripheral IV bolus of CVT-3146; Doppler signals were stable and interpretable over the time-course of the increase in CBFv in 17 patients.

RESULTS CVT-3146 caused a rapid increase in CBFv that was near peak by 30 to 40 seconds post onset of bolus. CVT-3146 at doses of 100 µg (n=3), 300 µg (n=4), and 500 µg (n=2) induced a peak to baseline ratio of 3.2 ± 0.6 (mean ± SD), similar to that obtained by IC Ado (3.2 ± 0.5). The duration of CBFv augmentation (≥ 2-fold increase in CBFv) was dose dependent; at 300 µg the duration was 4.0 ± 4.9 minutes and at 500 µg was 6.9 ± 7.6 minutes. At 500 µg (n=3) the maximal increase in heart rate (HR) was 18.7 ± 4.0 and the maximal decrease in systolic blood pressure (BP) was 8.7 ± 7.6. Adverse events (AEs) were infrequent and included nausea, flushing, and headache; these were mild and self-limited. No AEs were noted in the 3 patients receiving the 500 µg dose.

CONCLUSION: In humans peak CBFv following CVT-3146 (IV bolus) is comparable to CBFv following IC Ado without major changes in either HR or BP. This agent's magnitude and duration of effect, adverse event profile and bolus administration make CVT-3146 a useful pharmacological stress agent for myocardial perfusion imaging.

### EXAMPLE 2

This example is a study performed to determine the range of dosages over which the selective A_{2A} receptor agonist, CVT-3146 can be administered and be effective as a coronary vasodilator.

The study included patients undergoing a clinically indicated coronary catheterization with no more than a 70% stenosis in any coronary artery and no more than a 50% stenosis of the study vessel had CBFv determined by Doppler flow wire. Study subject were selected after measuring baseline and peak CBFv after an intracoronary (IC) injection of 18 µg of Ado. 36 subjects were identified as meeting the study criteria of having a peak to baseline CBFv ration of ≥ 2.5 in response to Adenosine,

CVT-3146 was administered to the study subjects by IV bolus in less that 10 seconds in amounts ranging from 10 µg to 500 µg.

The effectiveness of both compounds was measured by monitoring coronary flow velocity. Other coronary parameters that were monitored included heart rate and blood pressure. These parameters were measured in order to evaluate the time to peak dose response, the magnitude of the dose response and the duration of the dose response. Adverse events were also monitored. Coronary blood flow velocity was measured at the left anterior descending coronary artery (LAD) or left circumflex coronary artery (LCx). The velocity measurements were taken by following standard heart catheterization techniques and inserting a 0.014 inch Doppler-tipped Flowire into the LAD or LCx vessel and thereafter monitoring blood flow velocity. In addition, hemodynamic and electrocardiographic measurements were recorded continuously.

Overall, 36 human subjects (n=36) were evaluated. Of the 36, 18 were female and 18 were male. Their mean age was 53.4 and they ranged from 24-72 years in age. Of the 36 subjects evaluated, the LAD vessel of 31 subjects was monitored, and the LCx vessel of 5 subjects was monitored. The following doses (µg) of CVT-3146 were given to the subjects in a single iv bolus: 10 (n=4); 30 (n=6); 100 (n=4); 300 (n=7); 400 (n=9); 500 (n=6).

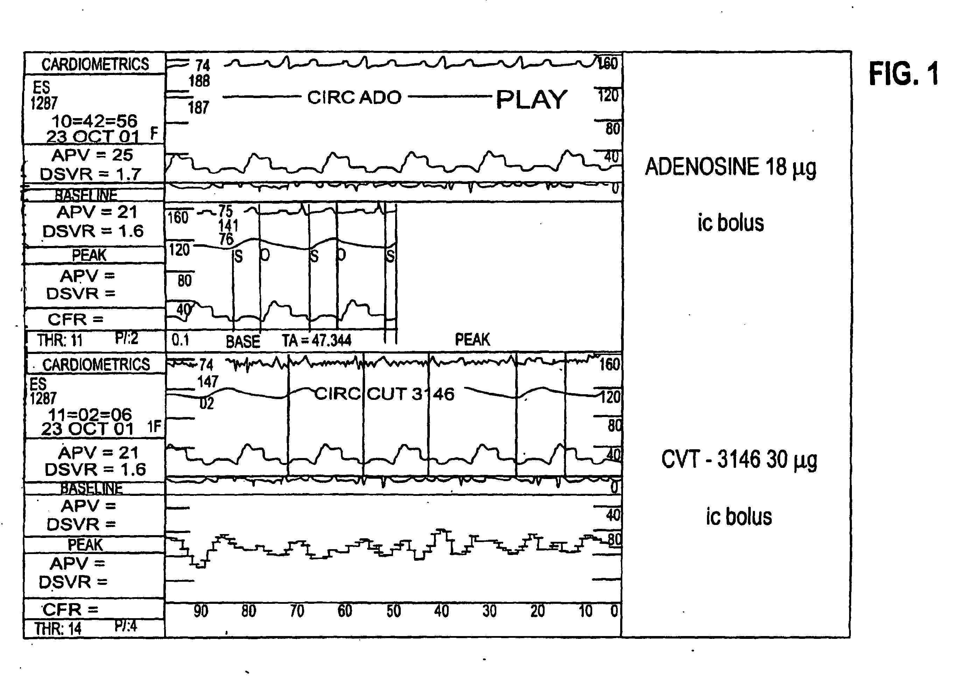

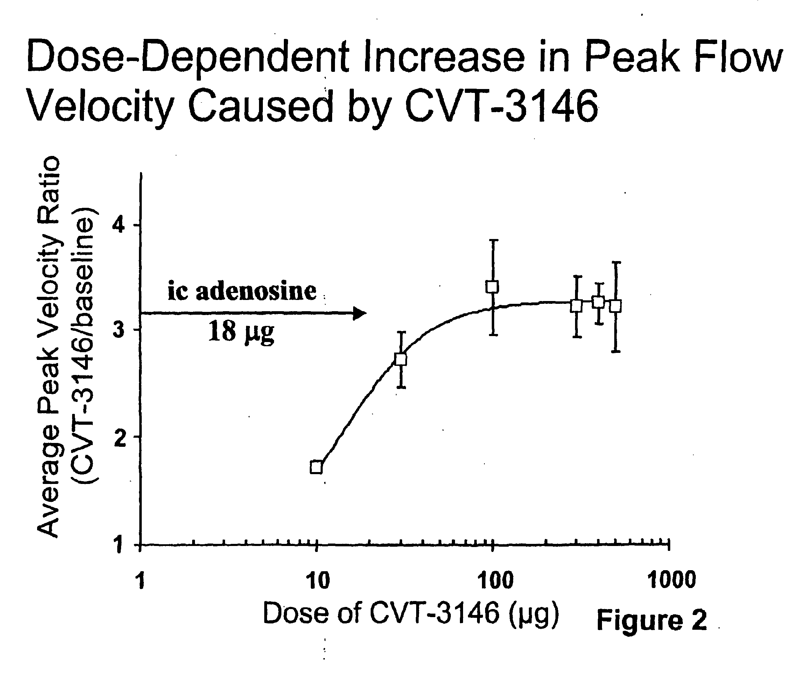

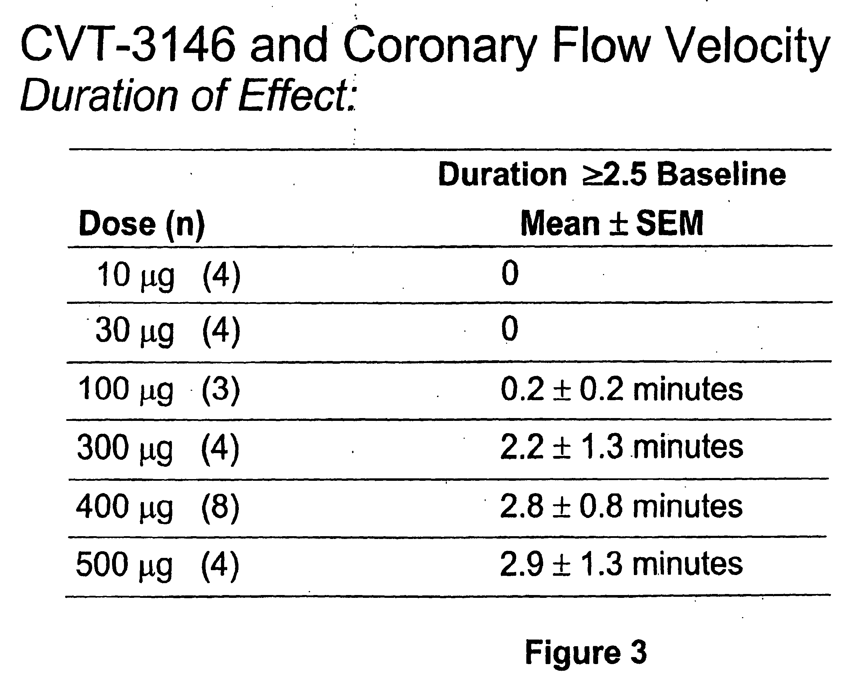

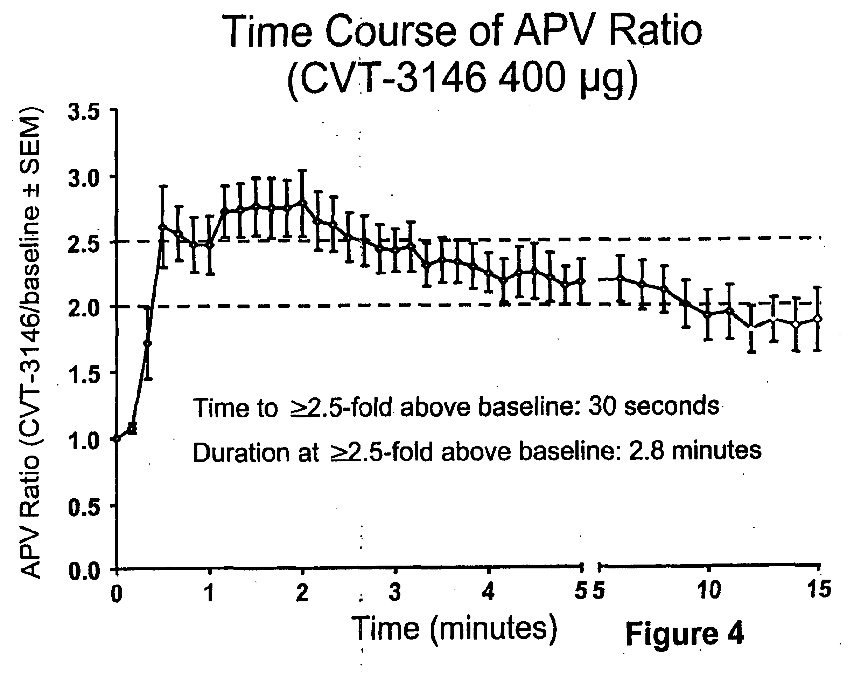

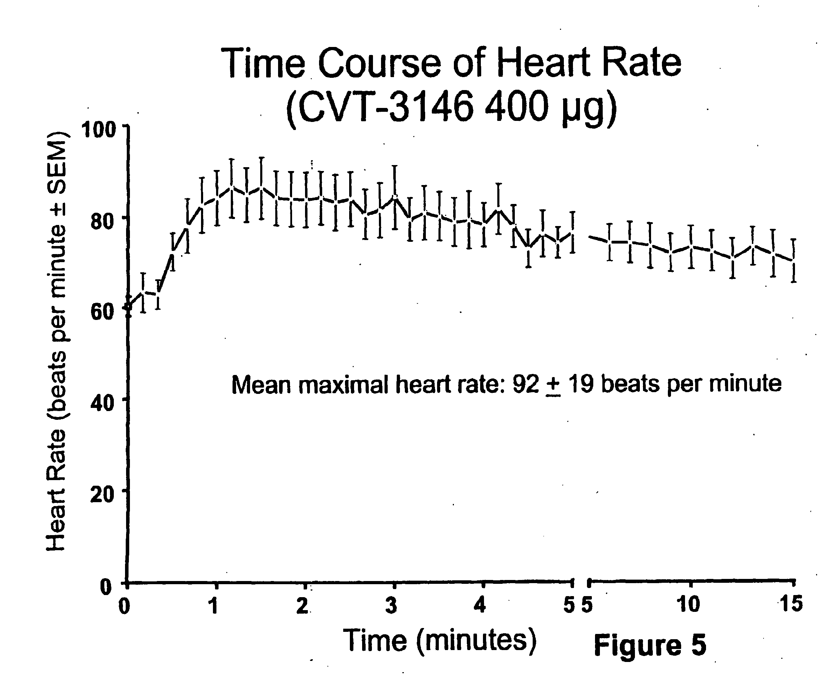

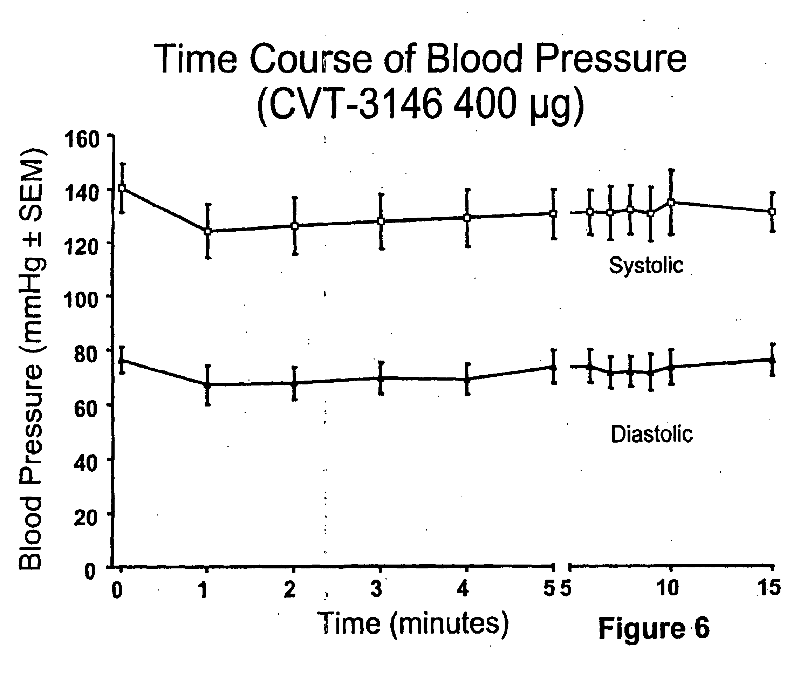

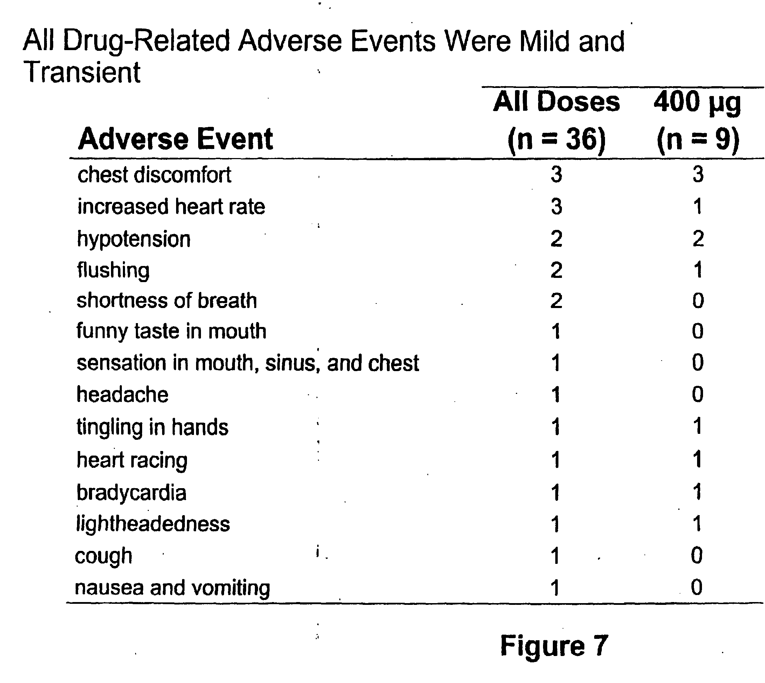

The study results are reported in Figures 1-6. The plot of Figure 1 shows that CVT-3146 increases peak flow velocity in amounts as low as 10 µg and reaches plateau peak velocity upon administration of less than about 100 µg of CVT-3146. Other test results and conclusions include:
- The peak flow was reached by about 30 seconds with all doses;
- At does above about 100 µg, peak effects were equivalent to 18 µg adenosine administered IC;
- CVT-3146 was generally well tolerated with adverse events being reported in The table attached as Figure 7;
- At 400 µg:
   o Coronary blood flow velocity ≥ 2.5-fold above baseline was maintained for 2.8 minutes.
   o Maximum increase in heart rate (18 ± 8 bpm) occurs about I minute after dosing.
   o Maximum decrease in systolic BP (20 ± 8 mmHg) occurs about 1 minute after dosing.
   o Maximum decrease in diastolic BP (10 ± 5 mmHg) occurs about 1 minute after dosing.

### Example 3

This Example is a study performed to evaluate (1) the maximum tolerated dose of CVT-3146 and (2) the pharmacokinetic profile of CVT-3146 in healthy volunteers, after a single IV bolus dose.

### Methods

The study was performed using thirty-six healthy, non-smoking male subjects between the ages of 18 and 59 and within 15% of ideal body weight.

### Study design

The study was performed in phase 1, single center, double-blind, randomized, placebo-controlled, crossover, ascending dose study. Randomization was to CVT-3146 or placebo, in both supine and standing positions.

CVT-3146 was administered as an IV bolus (20 seconds) in ascending doses of 0.1, 0.3, 1.3, 10, 20 and 30 µg/kg.

Subjects received either CVT-3146 or placebo on Day 1 supine, then crossover treatment on Day 2 supine. On Day 3, subjects received CVT-3146 or placebo standing, then crossover treatment on Day 4 standing.

### Assessments

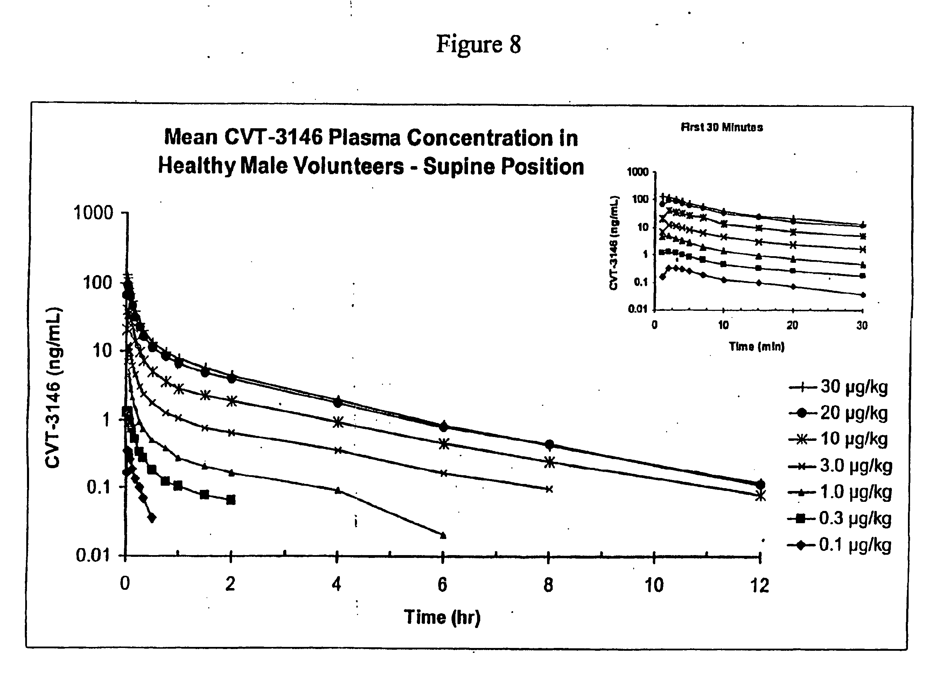

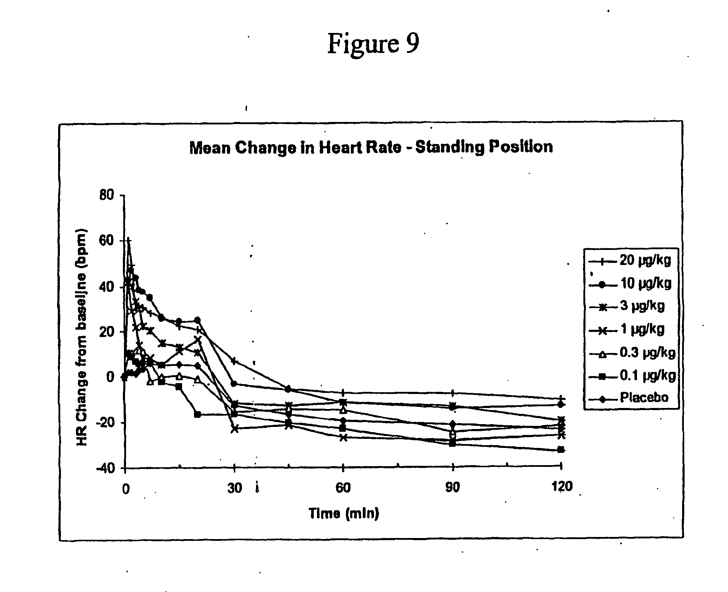

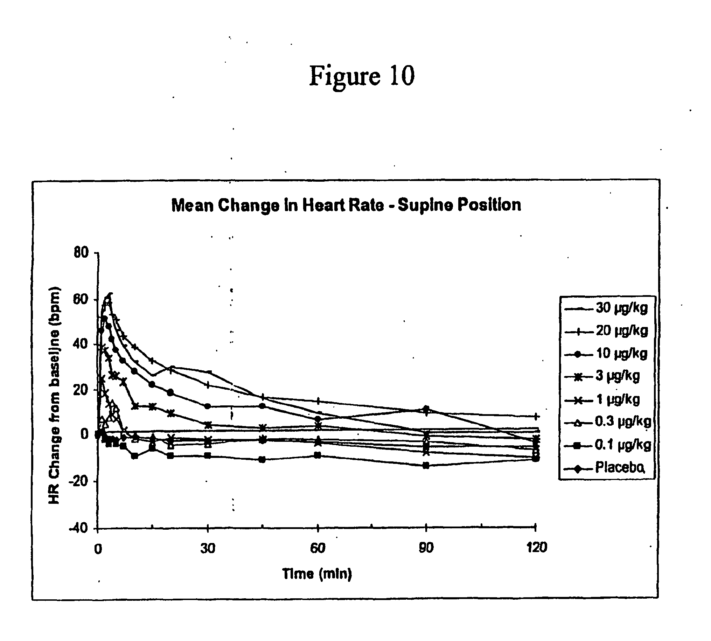

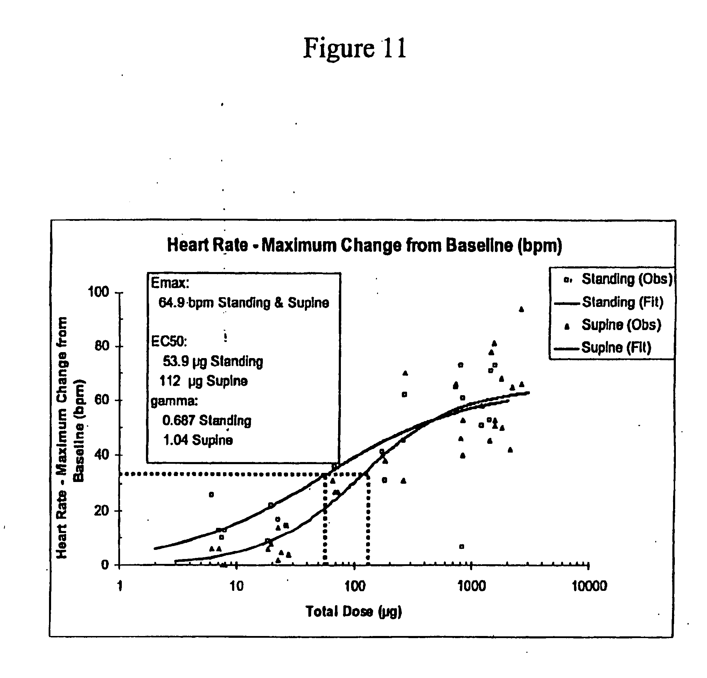

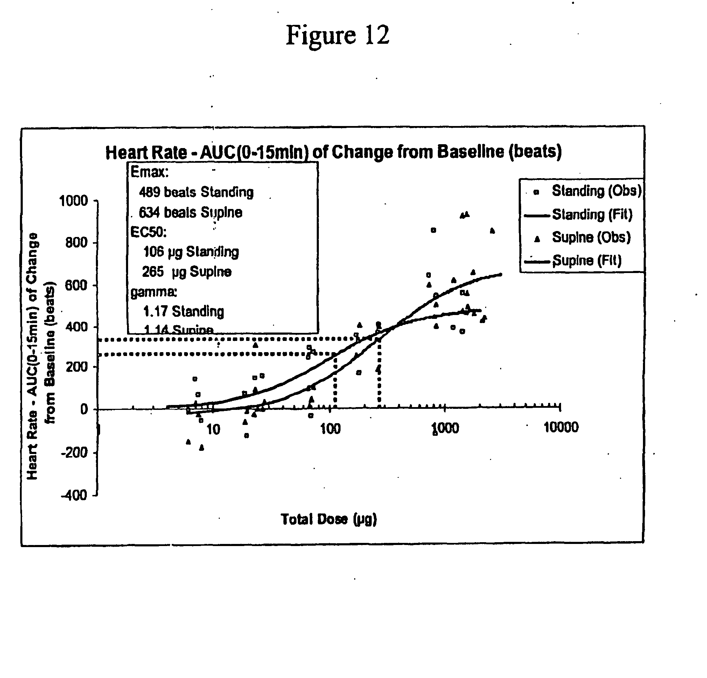

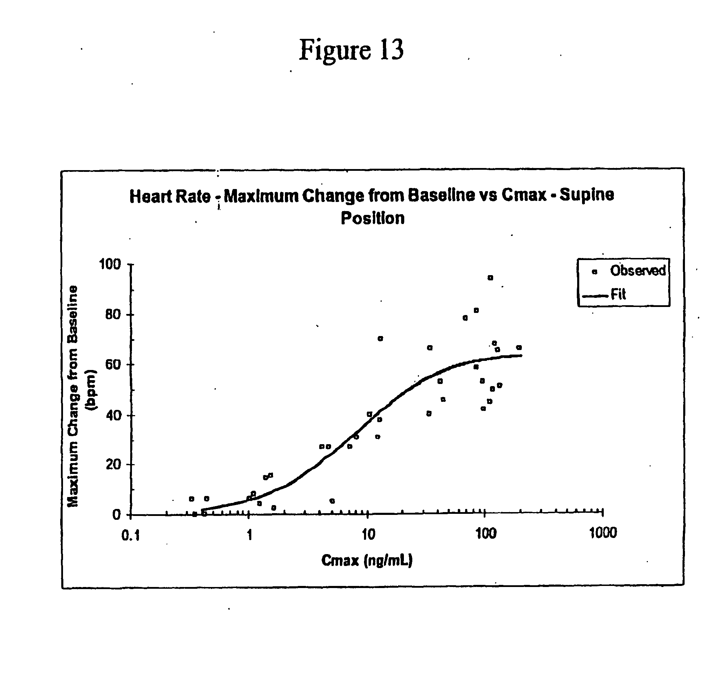

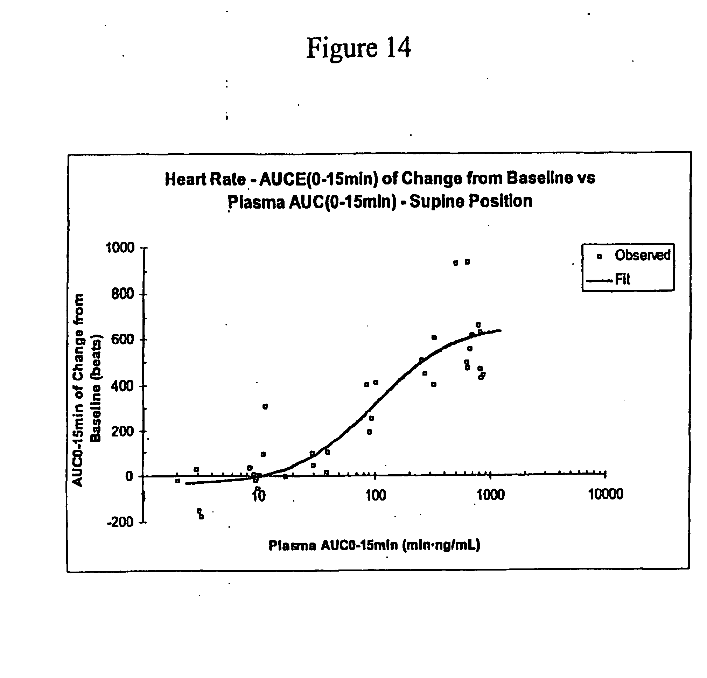

Patient safety was monitored by ECG, laboratory assessments, and collection of vital signs and adverse events.
Pharmacokinetics:
   Plasma samples were drawn during supine phase (Days 1 and 2) at 0, 1, 2, 3, 4, 5, 7, 10, 15, 20, 30, 45 minutes after dosing and at 1, 1.5, 2, 4, 6, 8,12 and 24 hours after dosing. Urine was collected for 24 hours for CVT-3146 excretion.
Pharmacodynamics:
   - The study evaluated the relationship of changes in heart rate to dose in both standing and supine positions and plasma concentration in the supine position. Some of the study results are reported in Figures 8-14.

### Results

### Safety

In general, adverse events reflected the pharmacologic effect of CVT-3146 and were related to vasodilation or an increase in heart rate (HR). Overall, adverse events were short-lived and mild to moderate in severity. There were no serious adverse events. Three events were assessed as severe in intensity (Table 1).

**Table 1. Adverse Events labeled as severe in intensity**

| | Number of Subjects with AE | |
|---|---|---|
| Event | 20 µg/kg Standing | 30 µg/kg Supine |
| No subjects per group | 4 | 4 |
| Palpitation | 0 | 2 |
| Dizziness | 1 | 0 |
| Syncope | 1 | 0 |

A three-compartment open model was fit to the data using observed Tmax (1-4 minutes) as the duration of a zero-order infusion. Reliable parameter estimates were obtained for dose of 1-30 µg/kg. Parameters are summarized in the following (Table 2):

**Table 2**

| Mean (SD) CVT-3146 Pharmacokinetic Parameters Estimated Using a Three - Compartment Model | | | | | | |
|---|---|---|---|---|---|---|
| Dose (µg(kg) | 1 | 3 | 10 | 20 | 30 | Total |
| N | 3 | 4 | 4 | 8 | 3 | 22 |
| CL (mL/min) | 737 (106) | 668 (167) | 841 (120) | 743 (123) | 1021 (92.7) | 768 (168) |
| Vc (L) | 9.84 (4.12) | 13.7 (6.06) | 17.9 (6.11) | 12.5 (5.83) | 15.7 (4.59) | 13.8 (5.67) |
| Vss (L) | 69.0 (28.2) | 90.0 (29.6) | 101 (11.3) | 75.2 (10.6) | 89.6 (10.9) | 75.5 (24.4) |
| α Half-life (min) | 2.14 (1.38) | 3.11 (2.14) | 4.15 (2.75) | 4.69 (4.01) | 3.00 (1.05) | 3.73 (2.88) |
| β Half-life (min) | 8.93 (4.10) | 17.2 (11.4) | 50.2 (52.1) | 32.6 (32.4) | 14.0 (4.98) | 27.2 (31.0) |
| λ Half-life (min) | 99.0 (28.6) | 130 (23.1) | 132 (20.5) | 117 (36.0) | 99.4 (8.10) | 86.4 (57.5) |
| K21 (1/min) | 0.246 (0.255) | 0.203 (0.272) | 0.187 (0.305) | 0.387 (0.615) | 0.0948 (0.0443) | 0.258 (0.410) |
| K31 (1/min) | 0.01808 (0.00548) | 0.0152 (0.00490) | 0.0108 (0.00592) | 0.0141 (0.00728) | 0.0148 (0.000900) | 0.0143 (0.00580) |

| | | | | | | |
|---|---|---|---|---|---|---|
| CL = clearance V_{c} = central volume of distribution Vₛₛ = volume of distribution at steady state K₂₁ = the rate constant for transfer from first peripheral to central compartment K₃₁ = rate constant for transfer from second peripheral to central compartment | | | | | | |

### Results

- CVT-3146 was well-tolerated, with AE's mainly representing its pharmacological effects as an adenosine A_{2A} receptor agonist.
- Mean tolerable dose for CVT-3146 was 10 µg/kg standing and 20 µg/kg supine.
- CVT-3146 does not require weight-adjusted dosing.
- There was no time lag between plasma concentration changes and changes in heart rate.
- The relationship between HR increase and dose or concentration was adequately described with a sigmoidal Emax model.

### Example 4

CVT-3146 is a novel selective A_{2A} adenosine receptor agonist being developed as a pharmacologic stressor for radionuclide myocardial perfusion imaging. Previously it has been shown that CVT-3146 causes coronary vasodilation without significantly affecting either total peripheral resistance or renal blood flow in awake dogs. The goal of this study was to determine the differential effects of CVT-3146 on blood flow velocity in various vascular beds.

The effect of CVT-3146 was studied on the blood flow velocity in left circumflex coronary artery (LCX), brain arterial vasculature (BA), forelimb artery (FA) and pulmonary artery (PA) of comparable diameter in the anesthetized dog. CVT-3146 (1.0 µg/kg) was administered as an intravenous bolus, transiently enhanced blood flow which was site specific. The effects of CVT-3146 were quantified as the average peak blood flow velocity (APV) using intravascular Doppler transducer tipped catheter. Heart rate (HR) and systemic arterial blood pressure (BP) were also monitored.

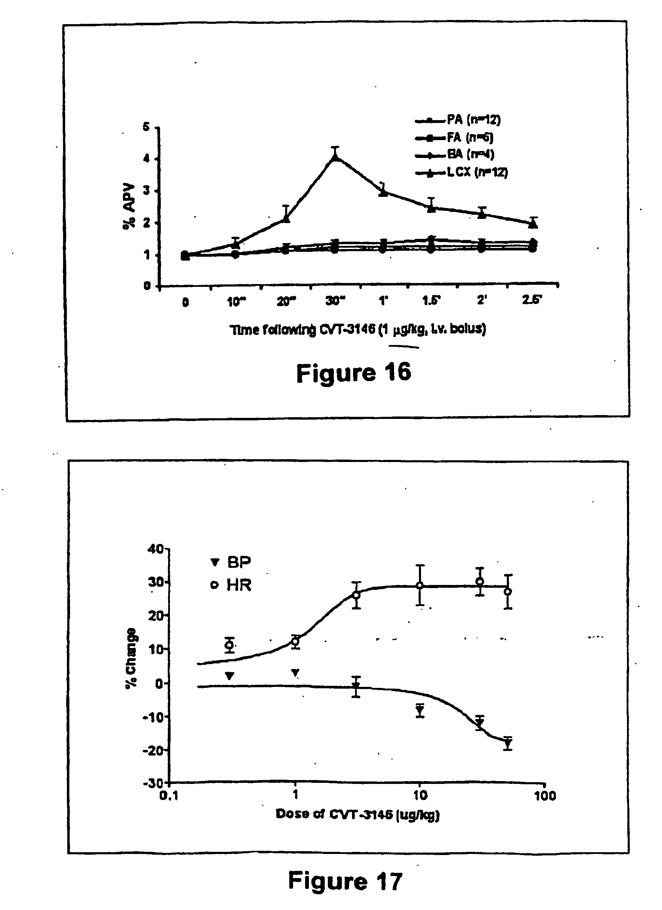

APV increased 3.1 ± 0.2, 1.4 ± 0,1, 1.2 ± 0.1, and 1.1 ± 0.01 fold in the LCX, BA, FA and PA, respectively manifesting a site-potency rank order of LCX>>BA>FA>PA (figure 16). The effect of CVT-3146 on blood flow velocity was short lasting; reaching a peak in less than 30 sec and dissipating in less than ten minutes. Increased blood flow velocity was associated with a small transient increase in HR (16 bpm) and decrease in BP (12 mmHg). In conclusion, this study demonstrated that CVT-3146 is a potent, short lasting vasodilator that is highly selective for the coronary vasculature.

### Example 5

The present study was carried out to determine whether CVT-3146, a selective A_{2A} adenosine receptor agonist, causes sympathoexcitation.
CVT-3146 (0.31 µg/kg-50 µg/kg) was given as a rapid i.v. bolus to awake rats and heart rate (HR) and blood pressure (BP) were monitored. CVT-3146 caused an increase in BP and systolic pressure (SP) at lower doses while at higher doses there was a decrease in BP and SP. CVT-3146 caused a dose-dependent increase in HR (Figure 17). The increase in HR was evident at the lowest dose of CVT-3146 at which there was no appreciable decrease in BP. ZM241385 (30 µg/kg, N=5), an A_{2A} receptor antagonist, attenuated the decrease in BP (CVT-3146: 14±3%, ZM: 1±1 %) and the increase in HR (CVT: 27±3%, ZM: 18±3%) caused by CVT-3146. Pretreatment with metoprolol (MET, 1 mg/kg, n=5), a beta-blocker, attenuated the increase in HR (CVT: 27±3%, MET: 15±2%), but had no effect on hypotension caused by CVT-3146. In the presence of hexamethonium (HEX, 10 mg/kg, n=5), a ganglionic blocker, the tachycardia was prevented (CVT: 27±3%, HEX: -1±2%), but BP was further reduced (CVT: -11±2%, HEX: -49±5%). CVT-3146 (10 µg/kg, n=6) also significantly (p<0.05) increased plasma norepinephrine (control: 146±11, CVT-3146:269±22 ng/ml) and epinephrine (control:25:f:5, CVT:100:f:20 ng/ml) levels. The separation ofHR and BP effects by dose, time and pharmacological interventions provides evidence that tachycardia caused by CVT-3146 is independent of the decrease in BP, suggesting that CVT-3146, via activation of A_{2A} receptors may cause a direct stimulation of the sympathetic nervous system.

### Example 6

Pharmacologic stress SPECT myocardial perfusion imaging (MPI) with adenosine (A) is a well-accepted technique, with excellent diagnostic and prognostic value and proven safety. However, side effects are common and AV nodal block and severe flushing are poorly tolerated. Agents such as CVT-3146 selectively act upon the A_{2A}-adenosine receptor and avoid stimulation of other receptor subtypes which may prevent such adverse reactions.

To determine the ability of CVT-3146 to produce coronary hyperemia and accurately detect CAD, 35 subjects (26 men, 9 women; 67±10 years) underwent both A and CVT-3146 stress/rest MPI, with 10.0±9.1 days between studies. Prior MI was noted in 12 patients, and many had prior revascularization [CABG (n=19),PCI (n=22)]. CVT-3146 [400 mcg (n=18), 500 mcg (n=17)] was administered as an IV bolus immediately followed by a saline flush, and then a Tc-99m radiopharmaceutical [sestamibi (n=34), tetrofosmin (n=1)]. SPECT images were uniformly processed, intermixed with control studies (normal and fixed-only defects), and interpreted by three observers in a blinded fashion using a 17-segment model. Quantitative analysis was also performed using 4D MSPECT. In addition to three separate readings, a consensus interpretation was performed and then a direct, same-screen comparison of A and CVT-3146 images undertaken to determine relative differences, using 5 regions per study.

The summed scores following stress were similar, both with visual (A=13.9±1.5, CVT-3146=13.2±1.3; p=n.s.) and quantitative methods of analysis (A=13.7±1.5, CVT - 3146=13.6±1.6; p=n.s.). Similarly, comparisons between the summed rest and summed difference scores were identical. The direct comparison also revealed no differences in ischemia detection, with a regional concordance for ischemia extent and severity of 86.3% and 83.4%, respectively. No dose-dependent effect of CVT-3146 on ischemia detection was noted. A conclusion of the study is that CVT-3146, administered by a logistically simple bolus injection, provides a similar ability to detect and quantify myocardial ischemia with SPECT MPI as noted with an A infusion.

### Example 7

CVT-3146 is a selective A_{2A} adenosine receptor agonist that produces coronary hyperemia and potentially less adverse effects due to its limited stimulation of receptor subtypes not involved with coronary vasodilation. This study evaluated the effectiveness of CVT-3146 as a pharmacologic stress agent.

36 subjects (27 men, 9 women; 67±10 years) were studied with two doses ofCVT-3146 [400 mcg (n=18), 500 mcg (n=18)], administered as an IV bolus, as part of a pharmacologic stress myocardial perfusion imaging protocol.

Adverse effects (AE) occurred in 26 pts (72%), including chest discomfort (33%), headache (25%), and abdominal pain (11%), with a similar incidence for both doses. Flushing, dyspnea, and dizziness were more frequent in the 500-mcg group (44%, 44%, and 28%, respectively) than in the 400-mcg group (17%, 17%, and 11 %, respectively). Most AEs were mild to moderate (96%) and resolved within 15 min without treatment (91%). One serious AE occurred, with exacerbation of a migraine headache, which required hospitalization. ST and T wave abnormalities developed with CVT-3146 in 7 and 5 pts, respectively. No 2nd or 3rd degree AV block was noted and there were no serious arrhythmias. Peak hemodynamic effects are shown in Table 3 and were noted at 4 min for systolic blood pressure (BP), 8 min for diastolic BP, and within 2 min for heart rate (HR). The effect on BP was minimal and systolic BP did not fall below 90 mmHg with either dose. The mean change in HR response was higher for the 500 mcg dose (44.2%) than for 400 mcg (34.8%; p=n.s.). Thirty min after CVT-3146, BP changes deviated <2% from baseline but HR remained above baseline by 8.6%.

The results of this study indicate that CVT-3146 is well-tolerated and has acceptable hemodynamic effects. Minimal differences were noted in BP and HR responses between the 400 mcg and 500 mcg doses, but AEs were more frequently at the higher dose. CVT-3146 appears safe and well-tolerated for bolus-mediated pharmacologic stress perfusion imaging. Hemodynamic Changes (mean±S.D.)

**Table 3**

| Absolute Change | Relative Change | |
|---|---|---|
| Heart Rate | +21.9±10.4 beats per min | +36.7%+21.0% |
| Systolic BP | -5.9±10.7 mmHg | -4.1%±7.6% |
| Diastolic BP | -5.4±7.2 mmHg | -7.9%±10.5% |

### Example 8

In this study the vasodilator effects of CVT-3146 were compared to those of ADO in different vascular beds in awake dogs. Dogs were chronically instrumented for measurements of the blood flow in coronary (CBF), mesenteric (MBF), hind limb (LBF), and renal (RBF) vascular beds, and hemodynamics. Bolus injections (iv) to CVT-3146 (0.1 to 2.5 µg/kg) and ADO (10 to 250 µg/kg) caused significant increases in CBF (35±6 to 205±23% and 58±13 to 163±16%) and MBF (18±4 to 88±14% and 36±8 to 84±5%).

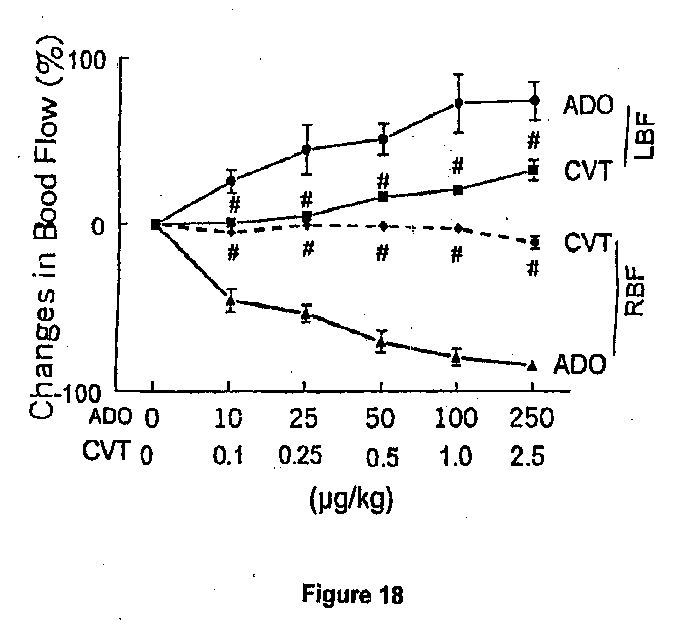

The results of the study demonstrate that CVT-3146 is a more potent and longer lasting coronary vasodilator compared to ADO (the duration for CBF above 2-fold of the baseline; CVT-3146 (2.5 µg/kg): 130±19s; ADO (250 µg/kg): 16±3s, P<0.5). As shown in Figure 18 (mean ± SE, n=6), CVT-3146 caused a smaller increase in LBF than ADO. ADO caused a dose-dependent renal vasoconstriction (RBF -46±7 to - 85±4%), whereas CVT-3146 has no or a little effect on RBF (-5±2 to -11±4%, P<0.05, compared to ADO). In conclusion, CVT-3146 is a more selective and potent coronary vasodilator than ADO. CVT-3146 has no the significant effect on renal blood flow in awake dogs. These features of CVT-3146 make it an ideal candidate for radionuclide myocardial perfusion imaging.

## Claims

1. A pharmaceutical composition comprising
a) the A₂ₐ receptor agonist named (1-{9-[(4S,2R,3R,5R)-3,4-dihydroxy-5-(hydroxymethyl)oxolan-2-yl]-6-aminopurin-2-yl}pyrazol-4-yl)-N-methylcarboxamide, which has the formula 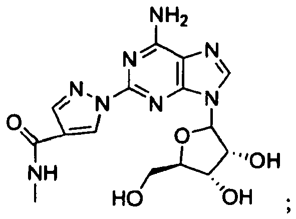
b) at least one liquid carrier selected from the group consisting of water, distilled water, de-ionized water, saline, a buffer, and combinations thereof;
c) propylene glycol in an amount from 5% to 25% (w:v); and
d) EDTA;
wherein the liquid carrier includes a buffer to bring said composition to a pH of from 6 to 8,
for use in a method of myocardial perfusion imaging of a human, said method comprising administering a radionuclide and the A₂ₐ receptor agonist in an amount of at least 400 µg, wherein the myocardium is examined for areas of insufficient blood flow following administration of the radionuclide and the A₂ₐ receptor agonist.

2. The pharmaceutical composition of claim 1 which is administered by iv bolus.

3. The pharmaceutical composition of claim 1, wherein the myocardium examination begins within about 1 minute from the time the A₂ₐ receptor agonist is administered.

4. The pharmaceutical composition of claim 1, wherein the administration of the A₂ₐ receptor agonist causes at least a 2.5 fold increase in coronary blood flow.

5. The pharmaceutical composition of claim 1, wherein the administration of the A₂ₐ receptor agonist causes at least a 2.5 fold increase in coronary blood flow that is achieved within about 1 minute from the administration of the A₂ₐ receptor agonist.

6. The pharmaceutical composition of claim 1, wherein the radionuclide and the A₂ₐ receptor agonist are administered separately.

7. The pharmaceutical composition of claim 1, wherein the radionuclide and the A₂ₐ receptor agonist are administered simultaneously.

8. The pharmaceutical composition of claim 1, wherein the administration of the A₂ₐ receptor agonist causes at least a 2.5 fold increase in coronary blood flow for less than about 5 minutes.

9. The pharmaceutical composition of claim 1, wherein the administration of the A₂ₐ receptor agonist causes at least a 2.5 fold increase in coronary blood flow for less than about 3 minutes.

10. The pharmaceutical composition of claim 1, wherein the A₂ₐ receptor agonist is administered in an amount of up to about 600 µg in a single dose.

11. The pharmaceutical composition of claim 1, wherein the A₂ₐ receptor agonist is administered in an amount of up to about 500 µg in a single dose.

12. The pharmaceutical composition of claim 1, wherein the A₂ₐ receptor agonist is administered in an amount of about 400 µg in a single dose.

## Patentansprüche

1. Pharmazeutische Zusammensetzung, die umfasst
a) den A₂ₐ-Rezeptor-Agonisten namens (1-{9-[(4S,2R,3R,5R)-3,4-Dihydroxy-5-(hydroxymethyl)oxolan-2-yl]-6-aminopurin-2-yl}pyrazol-4-yl)-N-methylcarboxamid, der die Formel 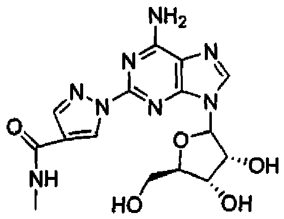 aufweist,
b) mindestens einen flüssigen Träger, ausgewählt aus der Gruppe, bestehend aus Wasser, destilliertem Wasser, deionisiertem Wasser, Salzlösung, einem Puffer und Kombinationen davon,
c) Propylenglykol in einer Menge von 5% bis 25% (w:v) und
d) EDTA,
wobei der flüssige Träger einen Puffer beinhaltet, um die Zusammensetzung auf einen pH-Wert von 6 bis 8 zu bringen,
zur Verwendung in einem Verfahren einer Myokardperfusionsdarstellung eines Menschen, wobei das Verfahren ein Verabreichen eines Radionuklids und des A₂ₐ-Rezeptor-Agonisten in einer Menge von mindestens 400 µg umfasst, wobei das Myokard hinsichtlich von Bereichen eines ungenügenden Blutflusses nach Verabreichung des Radionuklids und des A₂ₐ-Rezeptor-Agonisten untersucht wird.

2. Pharmazeutische Zusammensetzung nach Anspruch 1, die mittels iv-Bolus verabreicht wird.

3. Pharmazeutische Zusammensetzung nach Anspruch 1, wobei die Myokard-Untersuchung innerhalb von etwa 1 Minute von dem Zeitpunkt, an dem der A₂ₐ-Rezeptor-Agonist verabreicht wird, beginnt.

4. Pharmazeutische Zusammensetzung nach Anspruch 1, wobei die Verabreichung des A₂ₐ-Rezeptor-Agonisten mindestens eine 2,5-fache Zunahme des koronaren Blutflusses bewirkt.

5. Pharmazeutische Zusammensetzung nach Anspruch 1, wobei die Verabreichung des A₂ₐ,Rezeptor-Agonisten mindestens eine 2,5-fache Zunahme des koronaren Blutflusses bewirkt, die innerhalb von etwa 1 Minute von der Verabreichung des A₂ₐ-Rezeptor-Agonisten erreicht wird.

6. Pharmazeutische Zusammensetzung nach Anspruch 1, wobei das Radionuklid und der A₂ₐ-Rezeptor-Agonist separat verabreicht werden.

7. Pharmazeutische Zusammensetzung nach Anspruch 1, wobei das Radionuklid und der A₂ₐ-Rezeptor-Agonist gleichzeitig verabreicht werden.

8. Pharmazeutische Zusammensetzung nach Anspruch 1, wobei die Verabreichung des A₂ₐ-Rezeptor-Agonisten mindestens eine 2,5-flache Zunahme des koronaren Blutflusses für weniger als etwa 5 Minuten bewirkt.

9. Pharmazeutische Zusammensetzung nach Anspruch 1, wobei die Verabreichung des A₂ₐ-Rezeptor-Agonisten mindestens eine 2,5-face Zunahme des koronaren Blutflusses für weniger als etwa 3 Minuten bewirkt.

10. Pharmazeutische Zusammensetzung nach Anspruch 1, wobei der A₂ₐ-Rezeptor-Agonist in einer Menge von bis zu etwa 600 µg in einer Einzeldosis verabreicht wird.

11. Pharmazeutische Zusammensetzung nach Anspruch 1, wobei der A₂ₐ-Rezeptor-Agonist in einer Menge von bis zu etwa 500 µg in einer Einzeldosis verabreicht wird.

12. Pharmazeutische Zusammensetzung nach Anspruch 1, wobei der A₂ₐ-Rezeptor-Agonist in einer Menge von etwa 400 µg in einer Einzeldosis verabreicht wird.

## Revendications

1. Composition pharmaceutique comprenant :
a) l'agoniste du récepteur A₂ₐ dénommé (1-{9-[(4S,2R,3R,5R)-3,4-dihydroxy-5-(hydroxyméthyl)oxolane-2-yl]-6-aminopurine-2-yl}pyrazole-4-yl)-N-méthylcarboxamide, qui a la formule 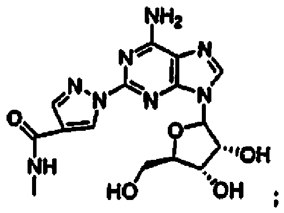
b) au moins un support liquide choisi dans le groupe consistant en eau, eau distillée, eau désionisée, solution saline, un tampon, et des combinaisons de ceux-ci;
c) du propylène glycol en une quantité de 5% à 25% (en poids: volume); et
d) de l'EDTA;
tandis que le support liquide comprend un tampon pour amener ladite solution à un pH de 6 à 8,
pour utilisation dans un procédé d'imagerie de perfusion myocardique d'un humain, ledit procédé comprenant l'administration d'un radionucléide et de l'agoniste du récepteur A₂ₐ en une quantité d'au moins 400 µg, tandis que le myocarde est examiné pour les zones de flux sanguin insuffisant après l'administration du radionucléide et de l'agoniste du récepteur A₂ₐ.

2. Composition pharmaceutique selon la revendication 1, qui est administrée par bolus iv.

3. Composition pharmaceutique selon la revendication 1, dans laquelle l'examen du myocarde commence dans un intervalle d'environ 1 minute à partir du moment auquel l'agoniste du récepteur A₂ₐ est administré.

4. Composition pharmaceutique selon la revendication 1, dans laquelle l'administration de l'agoniste du récepteur A₂ₐ provoque une augmentation d'au moins 2,5 fois du flux sanguin coronaire.

5. Composition pharmaceutique selon la revendication 1, dans laquelle l'administration de l'agoniste du récepteur A₂ₐ provoque une augmentation d'au moins 2,5 fois du flux sanguin coronaire qui est atteint dans un intervalle d'environ 1 minute à partir de l'administration de l'agoniste du récepteur A₂ₐ.

6. Composition pharmaceutique selon la revendication 1, dans laquelle le radionucléide et l'agoniste du récepteur A₂ₐ sont administrés séparément.

7. Composition pharmaceutique selon la revendication 1, dans laquelle le radionucléide et l'agoniste du récepteur A₂ₐ sont administrés simultanément.

8. Composition pharmaceutique selon la revendication 1, dans laquelle l'administration de l'agoniste du récepteur A₂ₐ provoque une augmentation d'au moins 2,5 fois du flux sanguin coronaire pendant moins d'environ 5 minutes.

9. Composition pharmaceutique selon la revendication 1, dans laquelle l'administration de l'agoniste du récepteur A₂ₐ provoque une augmentation d'au moins 2,5 fois du flux sanguin coronaire pendant moins d'environ 3 minutes.

10. Composition pharmaceutique selon la revendication 1, dans laquelle l'agoniste du récepteur A₂ₐ est administré en une quantité allant jusqu'à environ 600 µg en une seule dose.

11. Composition pharmaceutique selon la revendication 1, dans laquelle l'agoniste du récepteur A₂ₐ est administré en une quantité allant jusqu'à environ 500 µg en une seule dose.

12. Composition pharmaceutique selon la revendication 1, dans laquelle l'agoniste du récepteur A₂ₐ est administré en une quantité d'environ 400 µg en une seule dose.
